# EUROPÄISCHE PATENTSCHRIFT

(11) **EP 1 017 757 B1**
(45) Veröffentlichungstag und Bekanntmachung des Hinweises auf die Patenterteilung: **10.04.2002**
(21) Anmeldenummer: 98941391.9
(22) Anmeldetag: 21.07.1998
(51) Int. Cl.: C09K 19/02, C09K 19/46

(54) **FERROELEKTRISCHE FLÜSSIGKRISTALLANZEIGE MIT AKTIVEN MATRIX ELEMENTEN**
FERROELECTRIC LIQUID CRYSTAL DISPLAY PROVIDED WITH ACTIVE- MATRIX ELEMENTS
AFFICHEUR A CRISTAUX LIQUIDES FERRO-ELECTRIQUE DOTE D'ELEMENTS A MATRICE ACTIVE

(30) Priorität: 25.07.1997 DE 19732381
(43) Veröffentlichungstag der Anmeldung: 12.07.2000
(73) Patentinhaber: Aventis Research & Technologies GmbH & Co KG, 65926 Frankfurt am Main (DE)
(72) Erfinder: SCHMIDT, Wolfgang, D-51143 Köln (DE); DÜBAL, Hans-Rolf, D-65343 Eltville (DE)
(74) Vertreter: Isenbruck, Günter, Dr.
(86) Internationale Anmeldenummer: EP9804548
(87) Internationale Veröffentlichungsnummer: WO9905238

(56) Entgegenhaltungen:
- EP-A- 0 592 092
- EP-A- 0 694 599
- EP-A- 0 717 305
- WO-A-96/15092

## Beschreibung

Neben nematischen und cholesterischen Flüssigkristallen werden in jüngerer Zeit auch optisch aktive geneigt smektische (ferroelektrische) Flüssigkristalle in kommerziellen Displayvorrichtungen verwendet.

Clark und Lagerwall konnten zeigen, daß der Einsatz ferroelektrischer Flüssigkristalle (FLC) in sehr dünnen Zellen zu optoelektrischen Schalt- oder Anzeigeelementen führt, die im Vergleich zu den herkömmlichen TN ("twisted nematic")-Zellen um bis zu einem Faktor 1000 schnellere Schaltzeiten haben (siehe z. B. EP-A 0 032 362). Aufgrund dieser und anderer günstiger Eigenschaften, z. B. der bistabilen Schaltmöglichkeit und des nahezu blickwinkelunabhängigen Kontrasts, sind FLCs grundsätzlich für Anwendungsgebiete wie Computerdisplays gut geeignet.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Die einzelnen Bildelemente (Pixel) eines LC Displays sind üblicherweise in einer x,y Matrix angeordnet, die durch die Anordnung je einer Serie von Elektroden (Leiterbahnen) entlang der Reihen und der Spalten an der Unter- bzw. Oberseiteseite des Displays gebildet wird. Die Kreuzungspunkte der horizontalen (Reihen-) und vertikalen (Spalten-) Elektroden bilden adressierbare Pixel.

Diese Anordnung der Bildpunkte bezeichnet man üblicherweise als eine passive Matrix. Zur Adressierung wurden verschiedene Multiplex-Schemata entwickelt, wie beispielsweise in Displays 1993, Vol. 14, Nr. 2, S. 86-93 und Kontakte 1993 (2), S. 3-14 beschrieben. Die passive Matrixadressierung hat den Vorteil einer einfacheren Herstellung und damit verbundenen geringen Herstellkosten, jedoch den Nachteil, daß die passive Adressierung immmer nur zeilenweise erfolgen kann, was dazu führt, daß die Adressierungszeit des gesamten Bildschirms bei N Zeilen, das N-fache der Zeilenadressierungszeit beträgt. Bei üblichen Zeilenadressierungszeiten von ca. 50 Mikrosekunden bedeutet das eine Bildschirmadressierungszeit von ca. 60 Millisekunden bei z.B. HDTV Norm (High Definition TV, 1152 Zeilen), d.h. einer maximalen Bildfrequenz von ca. 16 Hz, zu langsam für bewegte Bilder. Zudem ist die Darstellung von Graustufen schwierig. Auf der FLC-Konferenz in Brest, Frankreich (20.-24 Juli 1997, siehe Abstract Book) wurde ein passives FLC Display mit digitalen Graustufen vorgestellt, bei dem jeder der RGB Bildpunkte (RGB= red, green, blue) in Unterpunkte unterteilt wurde, wodurch vermittels partiellem Schalten die Darstellung von Grauwerten in digitaler Form ermöglicht wird. Der Nachteil dieser Methode ist eine starke Erhöhung der Anzahl benötigter Bildschirmtreiber und damit der Kosten (im Fall des obigen Bildschirm werden dreimal soviele Treiber benötigt, wie bei einem normalen FLC Display ohne digitale Graustufen).

Bei der sogenannten Aktivmatrix-Technologie (AMLCD) wird üblicherweise ein nichtstrukturiertes Substrat mit einem Aktivmatrix-Substrat kombiniert. An jedem Pixel des Aktivmatrixsubstrates ist ein elektrisch nichtlineares Element, beispielsweise ein Dünnschichttransistor, integriert. Bei dem nichtlinearen Element kann es sich auch um Dioden, Metall-Insulator-Metall u.ä. Elemente handeln, die vorteilhaft mit Dünnschichtverfahren hergestellt werden und in der einschlägigen Literatur beschrieben sind (s. z.B. T. Tsukuda, TFT/LCD: Liquid Crystal Displays Addressed by Thin-Film Transistors, Gordon and Breach 1996, ISBN 2-919875-01-9 und darin zitierte Literatur).

Aktivmatrix-LCD werden üblicherweise mit nematischen Flüssigkristallen im TN- (twisted nematics), ECB- (electrically controlled birefringence), VA- (vertically aligned) oder IPS- (in plane switching) Modus betrieben. In jedem Fall wird durch die aktive Matrix an jedem Bildpunkt ein elektrisches Feld individueller Stärke erzeugt, das eine Orientierungsänderung und damit eine Änderung der Doppelbrechung erzeugt, die wiederum im polarisierten Licht optisch sichtbar ist. Ein schwerwiegender Nachteil dieser Verfahren ist die mangelnde Videofähigkeit, d.h. die zu langsamen Schaltzeiten nematischer Flüssigkristalle.

Unter anderem aus diesem Grunde wurden Flüssigkristallanzeigen, die auf der Kombination aus ferroelektrischen Flüssigkristallmaterialien und aktiven Matrix Elementen beruhen, z.B. in WO 97/12355, Ferroelectrics 1996, 179, 141-152 oder bei W.J.A.M. Hartmann (Dissertation, Eindhoven 1992) vorgeschlagen.

Im letzteren Fall wird eine Kombination aus der sogenannten 'Quasi-bookshelf Geometrie' (QBG) eines FLC und einer TFT (Thin-Film-Transistor) Aktivmatrix genutzt und damit gleichzeitig eine hohe Schaltgeschwindigkeit, Graustufen und hohe Transmission erzielt. Allerdings ist die QBG nicht über einen weiten Temperaturbereich stabil, da durch die Temperaturabhängigkeit der smektischen Schichtdicke die feldinduziert Lagenstruktur aufbricht oder sich dreht.

Die in kommerziellen FLC-Displays verwendete kommerzialisierte 'Chevron'-Geometrie (C1 oder C2) besitzt zwar eine hinreichend hohe Temperaturstabilität, jedoch keine ausreichende Helligkeit, da der effektive Tiltwinkel stark von seinem optimalen Wert entfernt liegt.

EP-A-0 694 599 und WO 96/15092 betreffen Fluorid enthaltende Flüssigkristallverbindungen für Passivmatrix-Flüssigkristallanzeigen.

EP-A-0 717 305 betrifft den Einsatz bestimmter ferroelektrischer Flüssigkristalle mit spontaner Polarisation in Aktivmatrix-Systemen.

Gegenstand der Erfindung ist ein Aktivmatrix-Flüssigkristalldisplay (LCD) enthaltend einen ferroelektrischen Flüssigkristall, der eine oder mehrere mesogene Verbindungen der nachstehenden Formel (I) enthält. Diese wird nicht wie bei der QBG durch Anlegen elektrischer Felder erzeugt und ist damit temperaturinstabil, sondern im Gegenteil über einen weiten Temperaturbereich stabil.

Die SBG wird erfindungsgemäß im Temperaturbereich der S_{c}*-Phase ausgebildet. Für eine Definition von "bookshelf" bzw. "spontaneous bookshelf" Geometrie siehe z.B. J.W. Goodby et al., Ferroelectric Liquid Crystals, Gordon & Breach, Philadelphia 1992, "Introduction to Ferroelectric Liquid Crystals; und Mizutani et al. in Conference Summaries, 6th Int. Conference on Ferroelectric Liquid Crystals, Brest, 1997, Seite 66.

Das erfindungsgemäße ferroelektrische Aktivmatrix-Flüssigkristalldisplay ist in hohem Maße praxistauglich, da es hohe Transmission, kurze Schaltzeit, Graustufen und einen weiten Temperaturbereich miteinander vereinbart.

Weiterhin weist das erfindungsgemäße Aktivmatrix-FLC-Display keine "zig-zag"-Deformationen auf, oder wenn, dann von derart geringer Ausprägung, daß sie nicht ins Gewicht fallen.

Erfindungsgemäß eingesetzte Displays, weisen eine weitgehend aufrechte, d. h. nicht geknickte ("non-chevron") smektische Lagenstruktur und einen hohen effektiven Tiltwinkel von mehr als 15° auf, und damit eine hohe Helligkeit und einen hohen Kontrast. Darüber hinaus können solche Displays vorteilhaft Elektrodenabstände von im allgemeinen 1 bis 4 µm, bevorzugt mindestens 1,5 µm, besonders bevorzugt mindestens 1,8 µm, aufweisen und lassen sich dennoch bei Spannungen von ≤ 40 Volt, bevorzugt ≤ 30 V schalten, besonders bevorzugt ≤ 15V, insbesondere ≤ 10 V.

Die spontane Polarisation des erfindungsgemäßen Aktiv-Matrix-FLCD liegt unterhalb 100 nC/cm², bevorzugt im Bereich von 0,1 bis 30 nC/cm², besonders bevorzugt 0,5 bis 15 nC/cm², insbesondere im Bereich von 1 bis 9 nC/cm² bei der Betriebstemperatur des Displays, d.h. vorzugsweise bei 30°C.
Bevorzugt sind insbesondere Aktiv-Matrix-FLCD mit einem 'layer-leaning angle' von kleiner als 10 °, einer Spontanpolarisation von von 1 bis 9 nC/cm², einem Schaltwinkel (gleich zweifacher Tiltwinkel) von 35-55 °, enthaltend mindestens zwei mesogene Strukturen der Formel (I).
Bevorzugt sind insbesondere auch poly-Silikon-Aktiv-Matrix-FLCD mit einem 'layer-leaning angle' von kleiner als 10°, einer Spontanpolarisation von von 0,1 bis 30 Nanocoulomb pro Quadratzentimeter, einem Schaltwinkel (gleich zweifacher Tiltwinkel) von 35-55 °, enthaltend mindestens zwei mesogene Strukturen der Formel (I).
Besonders bevorzugt sind Aktiv-Matrix-FLCD mit einem 'layer-leaning angle' von kleiner als 10 °, einer Spontanpolarisation von von 1 bis 9 Nanocoulomb pro Quadratzentimeter, einem Schaltwinkel (gleich zweifacher Tiltwinkel) von 35-55 °, enthaltend eine FLC Mischung mit mindestens 10 Komponenten, davon mindestens fünf mesogene Strukturen der Formel (I), die mindestens zusamengenommen einen Gewichtsanteil von 30 % an der Mischung ausmachen.
Mesogen bedeutet im Sinne der Erfindung, daß die Verbindung allein oder in Mischung mit anderen mesogenen Verbindungen eine Flüssigkristallphase, vorzugsweise eine ferroelektrische, ausbildet.
Erfindungsgemäß eingesetzte mesogene Verbindungen mit teil- oder perfluorierter Seitenkette sind solche der Formel (I),

R(-A¹-M¹)ₐ(-A²-M²)_{b}(-A³-M³)_{c}-A⁴-B-R_{f} (I)

in der die Symbole und Indizes folgende Bedeutungen haben:
- R: ist
a) Wasserstoff, -F, -Cl, -CF₃, -OCF₃ oder -CN,
b) ein geradkettiger oder verzweigter Alkylrest (mit oder ohne asymmetrisches C-Atom) mit 1 bis 20 C-Atomen, wobei
   b1) eine oder mehrere nicht benachbarte und nicht terminale CH2-Gruppen durch -O-, -S-, -CO-, -CO-O-, -O-CO-, -O-CO-O- oder -Si(CH₃)₂- ersetzt sein können und/oder
   b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
   b3) ein oder mehrere H-Atome durch F, CN und/oder Cl ersetzt sein können und/oder
   b4) die terminale CH₃-Gruppe durch eine der folgenden chiralen Gruppen (optisch aktiv oder racemisch) ersetzt sein kann: 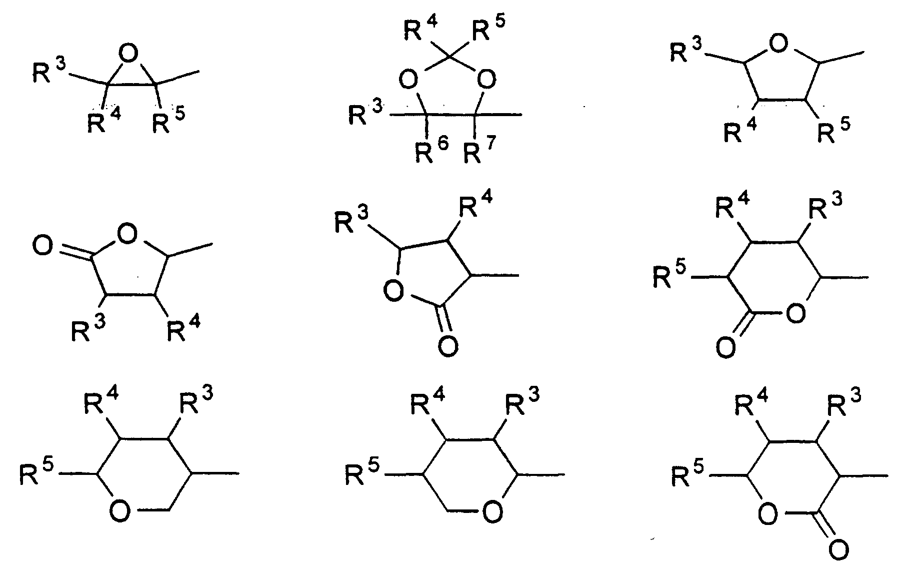 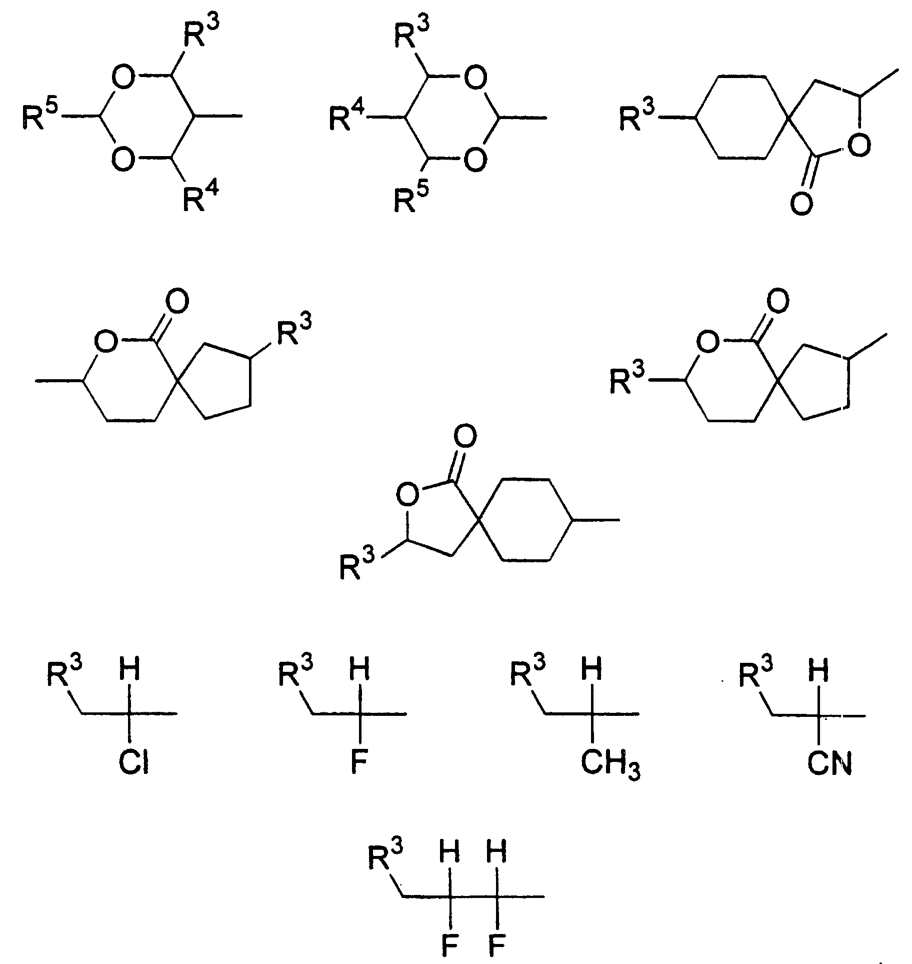
c) B-R_{f}.
- R³, R⁴, R⁵, R⁶, R⁷: sind gleich oder verschieden
a) Wasserstoff
b) ein geradkettiger oder verzweigter Alkylrest (mit oder ohne asymmetrisches Kohlenstoffatom) mit 1 bis 16 C-Atomen, wobei
   b1) eine oder mehrere nicht benachbarte und nicht terminale CH₂-Gruppen durch -O- ersetzt sein können und/oder
   b2) eine oder zwei CH₂-Gruppen durch -CH=CH- ersetzt sein können,
c) R⁴ und R⁵ zusammen auch -(CH₂)₄- oder -(CH₂)₅-, wenn sie an ein Oxiran-, Dioxolan-, Tetrahydrofuran-, Tetrahydropyran-, Butyrolacton- oder Valerolacton-System gebunden sind;
- R_{f}: ist
ein geradkettiger oder verzweigter teil- oder perfluorierter Alkylrest (mit oder ohne asymmetrisches C-Atom) mit 1 bis 20 C-Atomen, wobei
a) eine oder mehrere nicht benachbarte und nicht terminale CH₂- oder CF₂-Gruppen durch -O-, -S-, -CO-, -CO-O-, -O-CO-, -O-CO-O- oder -Si(CH₃)₂ersetzt sein können und/oder
b) eine oder mehrere CH₂- oder CF₂-Gruppen durch -CH=CH-, -C≡C-, Cyclopropan-1,2-diyl, 1,4-Phenylen, 1,4-Cyclohexylen oder 1,3-Cyclopentylen ersetzt sein können;
- B: ist
-O-, -S-, -(CH₂)ₙ₊₁-O-, -O-(CH₂)ₙ₊₁-, -(CH₂)ₙ₊₁-S-, -S-(CH₂)ₙ₊₁-, -CO-(CH₂)ₙ-, -CO-O-(CH₂)ₙ-, -O-CO-(CH₂)ₙ-, -CO-S-(CH₂)ₙ-, -S-CO-(CH₂)ₙ-, -CS-O-(CH₂)ₙ-, -O-CS-(CH₂)ₙ-, -SO₂-(CH₂)ₙ-, -OSO₂-(CH₂)ₙ-, -CH=CH-, -C≡C-, -(CH₂)ₙ₊₁-, -CH=N-, -N(CₖH₂ₖ₊₁)-, -(CH₂)ₙ-N(CₖH₂ₖ₊₁)-CO-, -(CH₂)ₙ-N(CₖH₂ₖ₊₁)-SO₂-, -O-[(CH₂)ₘ₊₁-O]ₗ-(CH₂)ₙ-, -[(CH₂)ₘ₊₁-O]ₗ-(CH₂)ₙ- oder eine Einfachbindung;
m, n sind gleich oder verschieden unabhängig voneinander eine ganze Zahl von 0 bis 15, k ist eine ganze Zahl von 0 bis 4 und I ist eine ganze Zahl von 1 bis 6, mit der Maßgabe, daß m+n ≤ 15 ist;
- M¹, M², M³: sind gleich oder verschieden
-CO-O-, -O-CO-, -CO-S-, -S-CO-, -CS-O-, -O-CS-, -CS-S-, -S-CS-, -CH₂-O-, -O-CH₂-, -CH₂-S-, -S-CH₂-, -CH₂-CH₂-, -CH=CH-, -C≡C-, -CH₂-CH₂-CO-O-, -O-CO-CH₂-CH₂-, -CH=N- oder eine Einfachbindung;
- A¹, A², A³, A⁴: sind gleich oder verschieden
1,4-Phenylen, wobei ein oder mehrere H-Atome durch F, Cl, CH₃, C₂H₅, OCH₃, CF₃, OCF₃ und/oder CN ersetzt sein können, 1,3-Phenylen, wobei eine oder zwei CH-Gruppen durch N ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, 1,4-Cyclohexylen, wobei ein oder zwei H-Atome durch CN und/oder CH₃ und/oder F ersetzt sein können, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F, Cl und/oder CN ersetzt sein kann, 1,3-Thiazol-2,5-diyl, wobei ein H-Atom durch F, Cl und/oder CN ersetzt sein kann, Thiophen-2,4-diyl, wobei ein H-Atom durch F, Cl und/oder CN ersetzt sein kann, Thiophen-2,5-diyl, wobei ein oder zwei H-Atome durch F, Cl und/oder CN ersetzt sein können, Naphthalin-2,6-diyl, Naphtalin-1,4-diyl oder Naphtalin-1,5-diyl, wobei jeweils ein oder mehrere H-Atome durch F, Cl und/oder CN ersetzt sein können und/oder eine oder zwei CH-Gruppen durch N ersetzt sein können, Phenanthren-2,7-diyl oder 9,10-Dihydrophenanthren-2,7-diyl, wobei jeweils ein, zwei oder mehrere H-Atome durch F ersetzt sein können und/oder eine oder zwei CH-Gruppen durch N ersetzt sein können, Indan-2,5-diyl, Indan-1-on-2,5-diyl, Benzothiazol-2,6-diyl, Benzothiazol-2,5-diyl, Benzoxazol-2,6-diyl, Benzoxazol-2,5-diyl, Benzofuran-2,5-diyl, Benzofuran-2,6-diyl, 2,3-Dihydrobenzofuran-2,5-diyl, Piperazin-1,4-diyl, Piperazin-2,5-diyl, 1-Alkyl-1-silacyclohexylen-1,4-diyl oder 1,3-Dioxaborinan-2,5-diyl;
- a, b, c: sind null oder eins,
mit der Maßgabe, daß die Verbindung der Formel (I) nicht mehr als vier fünf- oder mehrgliedrige Ringsysteme enthalten darf.

Bevorzugt haben die Symbole und Indizes in der Formel (I) folgende Bedeutungen:
- R: ist bevorzugt gleich oder verschieden
a) Wasserstoff, -F, -OCF₃, oder -CN,
b) ein geradkettiger oder verzweigter Alkylrest (mit oder ohne asymmetrisches C-Atom) mit 1 bis 18 C-Atomen, wobei
   b1) eine oder mehrere nicht benachbarte und nicht terminale CH₂-Gruppen durch -O-, -CO-O-, -O-CO-, -O-CO-O- oder -Si(CH₃)₂ersetzt sein können und/oder
   b2) eine CH₂-Gruppe durch Cyclopropan-1,2-diyl, 1,4-Phenylen oder trans-1,4-Cyclohexylen ersetzt sein kann und/oder
   b3) ein oder mehrere H-Atome durch F ersetzt sein können und/oder
   b4) die terminale CH₃-Gruppe durch eine der folgenden chiralen Gruppen (optisch aktiv oder racemisch) ersetzt sein kann: 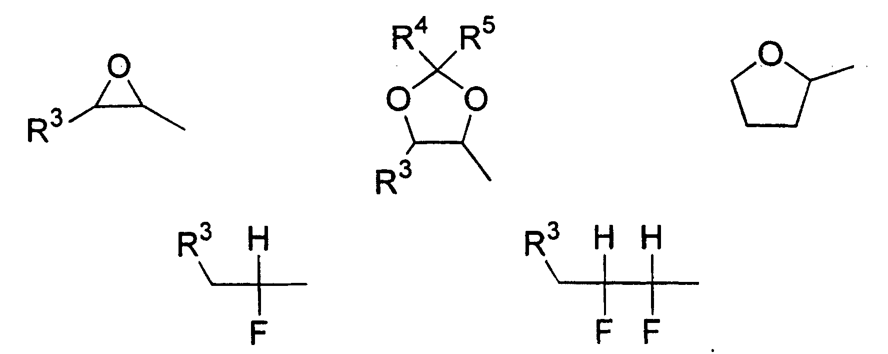
- R: ist besonders bevorzugt gleich oder verschieden
a) Wasserstoff,
b) ein geradkettiger oder verzweigter Alkylrest (mit oder ohne asymmetrisches C-Atom) mit 1 bis 16 C-Atomen, wobei
   b1) eine oder zwei nicht benachbarte und nicht terminale CH₂-Gruppen durch -O-, -CO-O-, -O-CO-, -O-CO-O- oder -Si(CH₃)₂ersetzt sein können und/oder
   b2) eine CH₂-Gruppe durch 1,4-Phenylen oder trans-1,4-Cyclohexylen ersetzt sein kann und/oder
   b3) ein oder mehrere H-Atome durch F ersetzt sein können und/oder
   b4) die terminale CH₃-Gruppe durch eine der folgenden chiralen Gruppen (optisch aktiv oder racemisch) ersetzt sein kann: 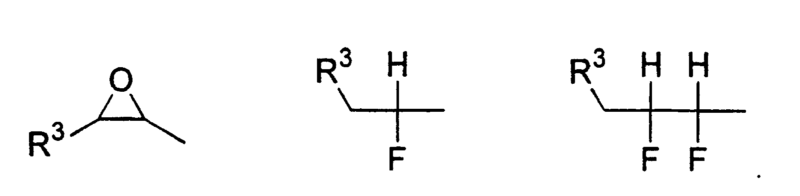
- R³, R⁴, R⁵, R⁶, R⁷: sind bevorzugt gleich oder verschieden
a) Wasserstoff,
b) ein geradkettiger oder verzweigter Alkylrest (mit oder ohne asymmetrisches Kohlenstoffatom) mit 1 bis 14 C-Atomen, wobei
   b1) eine oder zwei nicht benachbarte und nicht terminale CH₂-Gruppen durch -O- ersetzt sein können und/oder
   b2) eine CH₂-Gruppe durch -CH=CH- ersetzt sein kann,
c) R⁴ und R⁵ zusammen auch -(CH₂)₄- oder -(CH₂)₅-, wenn sie an ein Oxiran-, Dioxolan-, Tetrahydrofuran-, Tetrahydropyran-, Butyrolacton- oder Valerolacton-System gebunden sind.
- R³, R⁴, R⁵, R⁶, R⁷: sind besonders bevorzugt gleich oder verschieden
a) Wasserstoff,
b) ein geradkettiger oder verzweigter Alkylrest (mit oder ohne asymmetrisches Kohlenstoffatom) mit 1 bis 14 C-Atomen, wobei
   b1) eine nicht terminale CH₂-Gruppe durch -O- ersetzt sein kann,
c) R⁴ und R⁵ zusammen auch -(CH₂)₄- oder -(CH₂)₅-, wenn sie an ein Oxiran-, Dioxolan-, Tetrahydrofuran-, Tetrahydropyran-, Butyrolacton - oder Valerolacton-System gebunden sind.
- R_{f}: ist bevorzugt
ein geradkettiger teil- oder perfluorierter Alkylrest mit 2 bis 18 C-Atomen, wobei
a) eine oder mehrere nicht benachbarte und nicht terminale CH₂- oder CF₂-Gruppen durch -O-, -CO-O- oder -O-CO- ersetzt sein können und/oder
b) eine CH₂- oder CF₂-Gruppe durch -CH=CH-, -C≡C-, Cyclopropan-1,2-diyl, 1,4-Phenylen, *trans*-1,4-Cyclohexylen oder 1,3-Cyclopentylen ersetzt sein kann.
- R^{f}: ist besonders bevorzugt
ein geradkettiger perfluorierter Alkylrest mit 4 bis 16 C-Atomen, wobei
a) eine oder zwei nicht benachbarte und nicht terminale CF₂-Gruppen durch -O- ersetzt sein können.
- B: ist bevorzugt
-O-, -(CH₂)ₙ₊₁-O-, -O-(CH₂)ₙ₊₁-, -CO-O-(CH₂)ₙ-, -O-CO-(CH₂)ₙ-, -CH=CH-, -C≡C-, -(CH₂)ₙ₊₁-, -O-[(CH₂)ₘ₊₁-O]ₗ-(CH₂)ₙ-, -[(CH₂)ₘ₊₁-O]ₗ-(CH₂)ₙ- oder eine Einfachbindung.
- B: ist besonders bevorzugt
-O-, -O-(CH₂)ₙ-, -CO-O-(CH₂)ₙ₋₁-, -O-CO-(CH₂)ₙ₋₁-, -(CH₂)ₙ-, -O-[(CH₂)ₘ₊₁-O]ₗ-(CH₂)ₙ- oder eine Einfachbindung.
- M¹, M², M³: sind bevorzugt gleich oder verschieden
-CO-O-, -O-CO-, -CH₂-O-, -O-CH₂-, -CH=CH-, -C≡C-, -CH₂-CH₂-CO-O-, -O-CO-CH₂-CH₂- oder eine Einfachbindung.
- M¹, M², M³: sind besonders bevorzugt gleich oder verschieden
-CO-O-, -O-CO-, -CH₂-O-, -O-CH₂- oder eine Einfachbindung.
- A¹, A², A³, A⁴: sind bevorzugt gleich oder verschieden
1,4-Phenylen, wobei ein oder zwei H-Atome durch F, Cl, CH₃ und/oder CN ersetzt sein können, 1,3-Phenylen, Pyridin-2,5-diyl, wobei ein oder zwei H-Atome durch F und/oder CN ersetzt sein können, Pyrimidin-2,5-diyl, wobei ein oder zwei H-Atome durch F und/oder CN ersetzt sein können, trans-1,4-Cyclohexylen, wobei ein oder zwei H-Atome durch CN und/oder F ersetzt sein können, (1,3,4)-Thiadiazol-2,5-diyl, 1,3-Dioxan-2,5-diyl, 1,3-Thiazol-2,4-diyl, wobei ein H-Atom durch F ersetzt sein kann, 1,3-Thiazol-2,5-diyl, wobei ein H-Atom durch F ersetzt sein kann, Thiophen-2,5-diyl, wobei ein oder zwei H-Atome durch F ersetzt sein können, Naphthalin-2,6-diyl, wobei jeweils ein oder zwei H-Atome durch F und/oder CN ersetzt sein können und/oder eine oder zwei CH-Gruppen durch N ersetzt sein können, Phenanthren-2,7-diyl oder 9,10-Dihydrophenanthren-2,7-diyl, wobei jeweils ein, zwei oder mehrere H-Atome durch F ersetzt sein können und/oder eine oder zwei CH-Gruppen durch N ersetzt sein können, Indan-2,5-diyl, Benzothiazol-2,6-diyl oder Benzothiazol-2,5-diyl.
- A¹, A², A³, A⁴: sind besonders bevorzugt gleich oder verschieden
1,4-Phenylen, wobei ein oder zwei H-Atome durch F, Cl und/oder CN ersetzt sein können, 1,3-Phenylen, Pyridin-2,5-diyl, wobei ein H-Atom durch F ersetzt sein kann, Pyrimidin-2,5-diyl, trans-1,4-Cyclohexylen, wobei ein oder zwei H-Atome durch CN und/oder F ersetzt sein können, (1,3,4)-Thiadiazol-2,5-diyl, 1,3-Thiazol-2,5-diyl, Thiophen-2,5-diyl, Naphthalin-2,6-diyl, wobei eine oder zwei CH-Gruppen durch N ersetzt sein können, Phenanthren-2,7-diyl oder 9,10-Dihydrophenanthren-2,7-diyl, wobei jeweils ein oder zwei H-Atome durch F ersetzt sein können und/oder eine oder zwei CH-Gruppen durch N ersetzt sein können oder Indan-2,5-diyl.

Ganz besonders bevorzugte Verbindungen der Formel (I) sind solche der Formeln (I-1) bis (I-64): 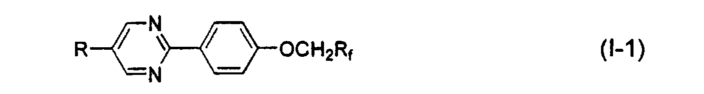 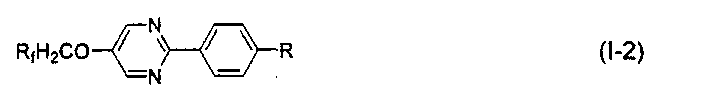 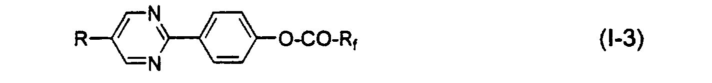 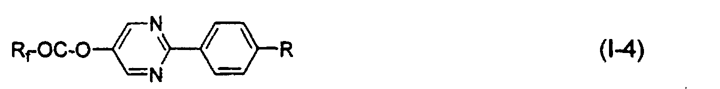 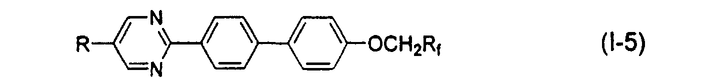 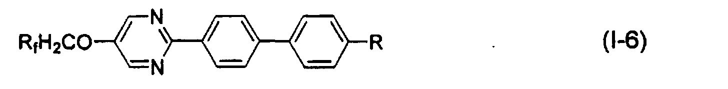 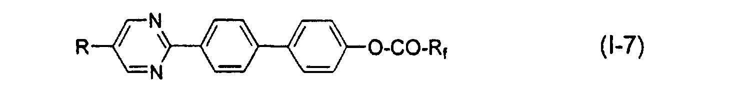 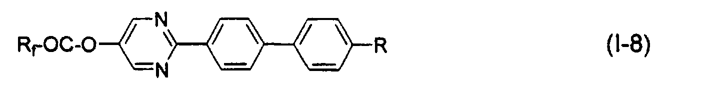 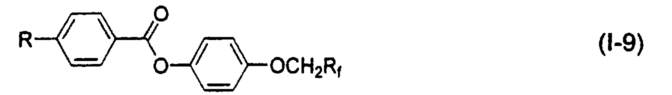 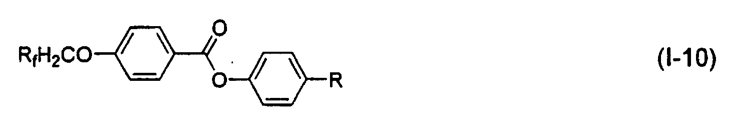 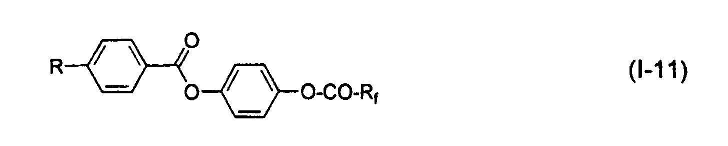 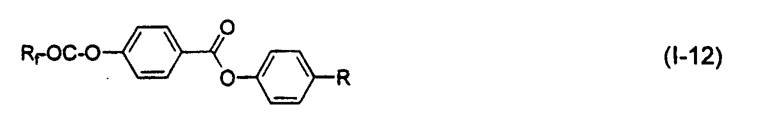 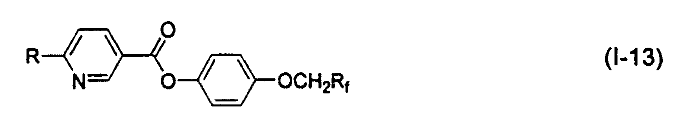 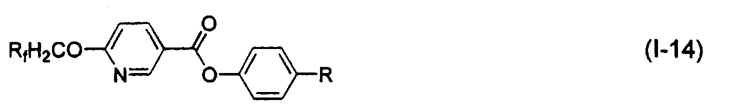 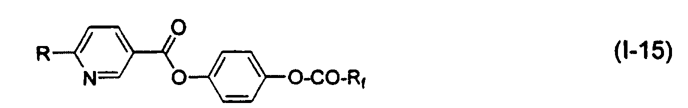 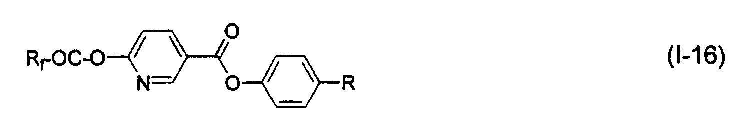 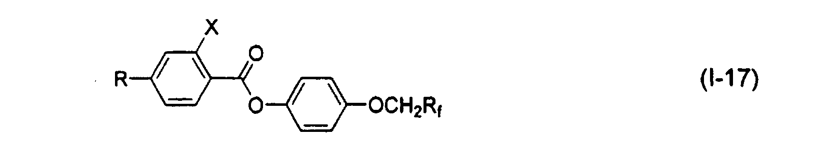 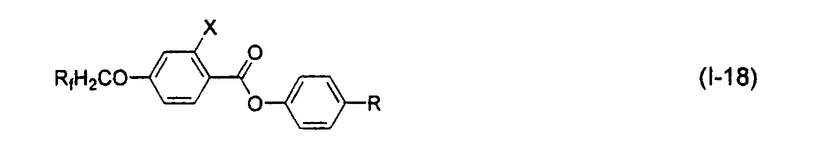 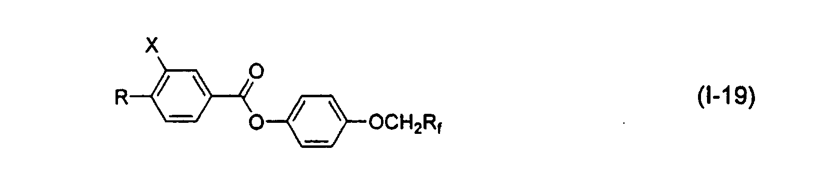 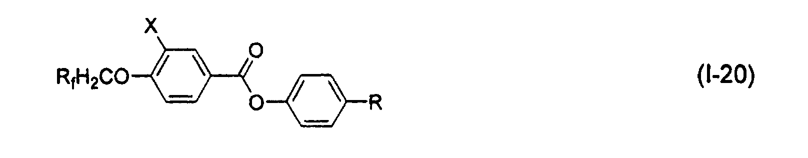 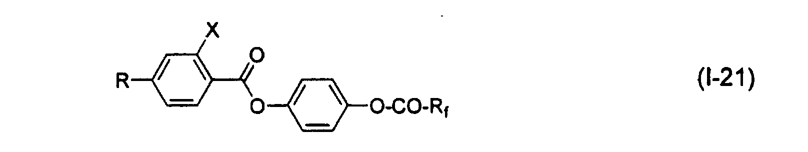 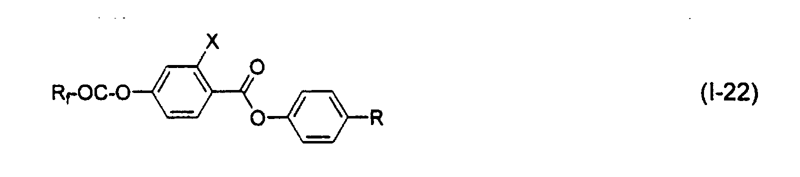 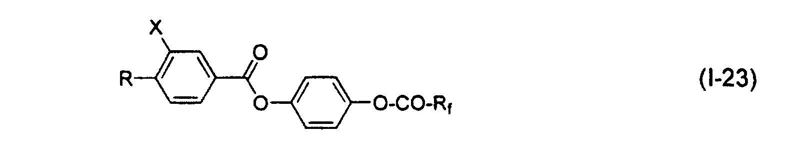 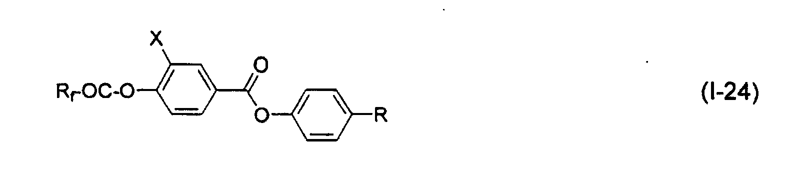 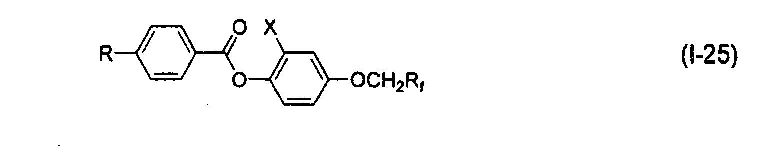 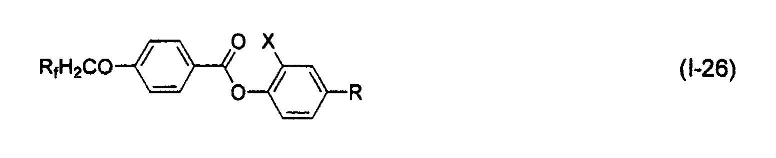 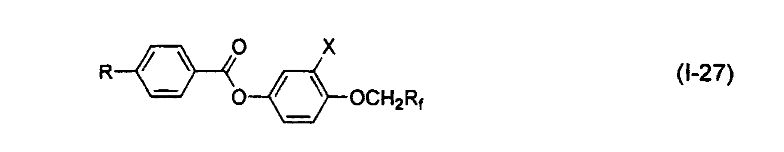 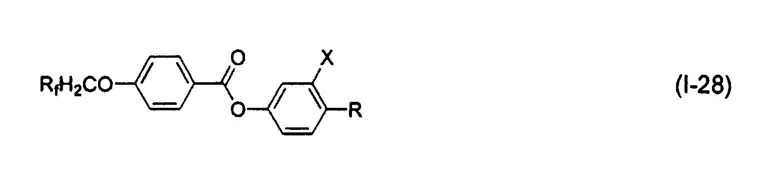 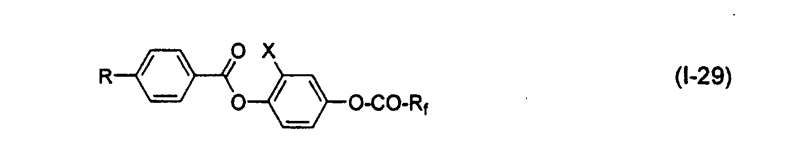 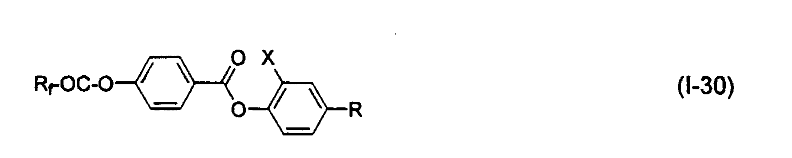 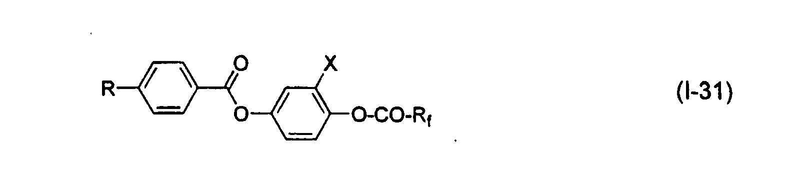 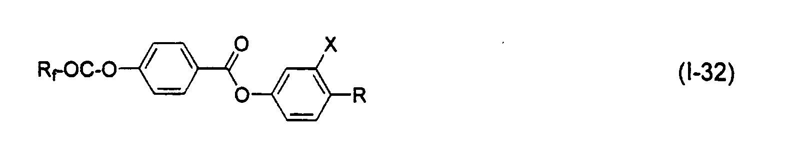 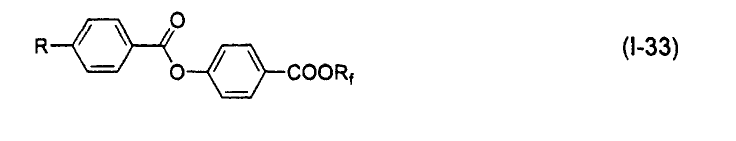 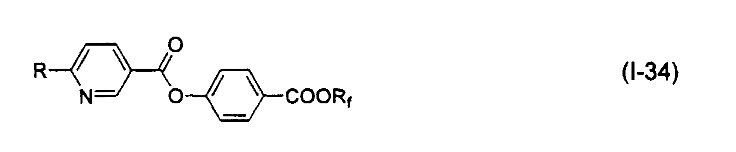 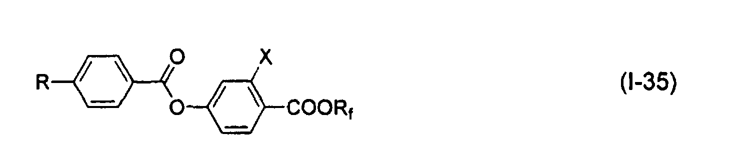 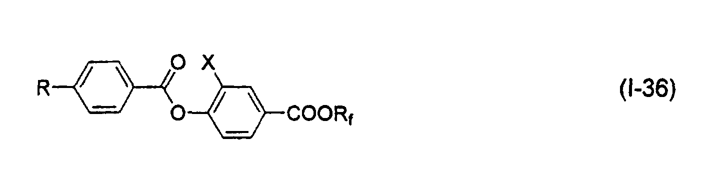 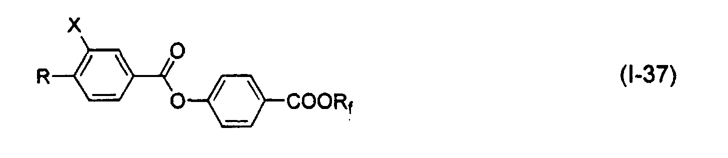 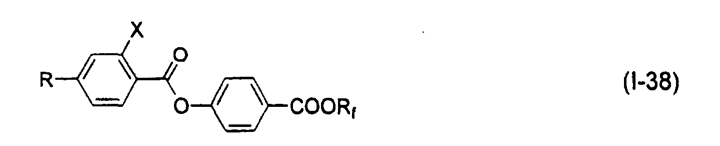 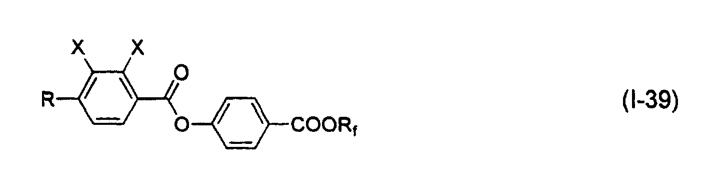 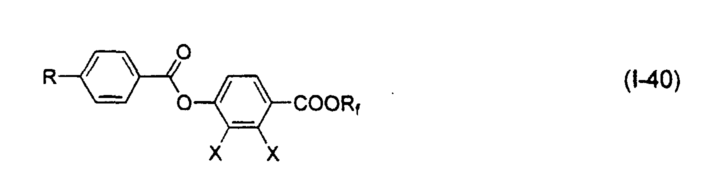 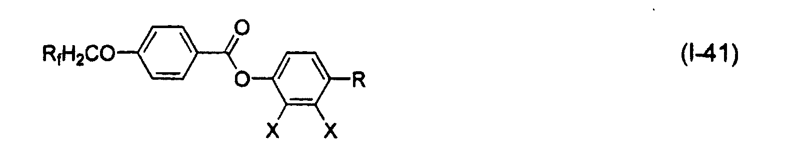 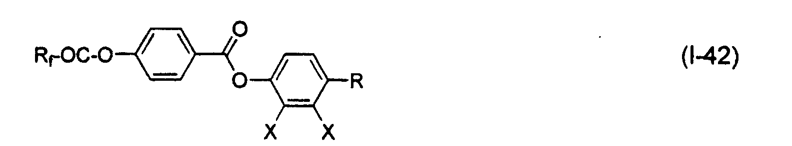 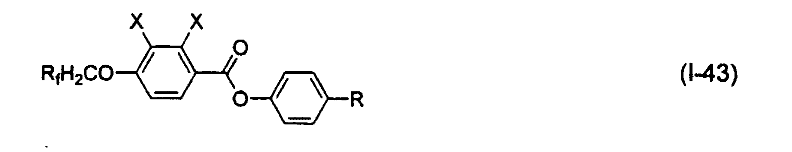 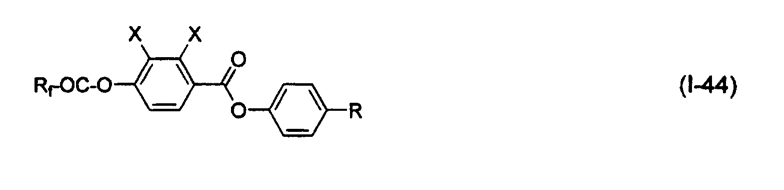 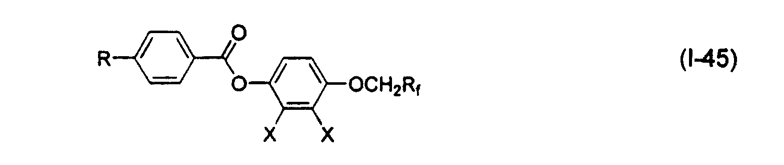 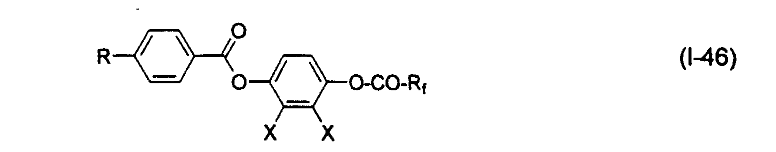 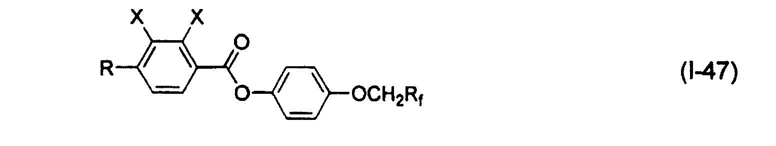 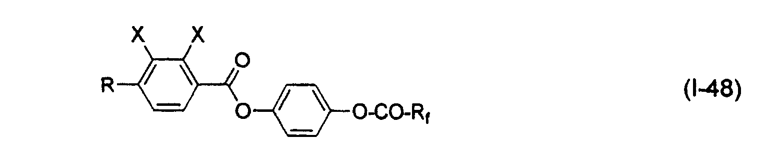 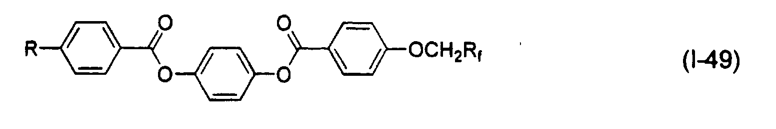 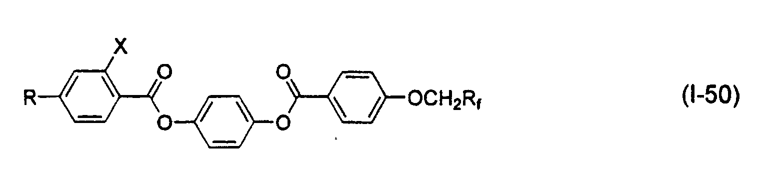 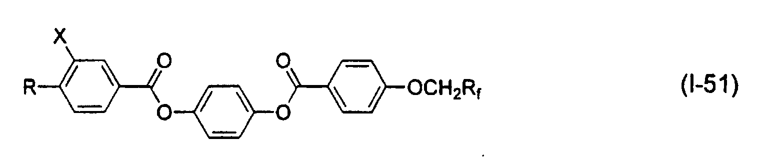 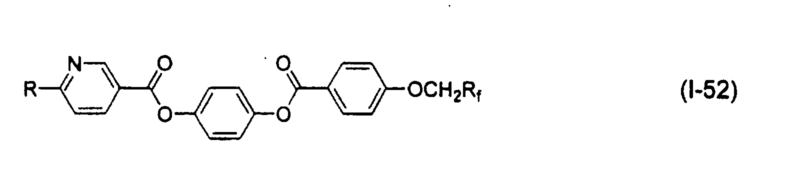 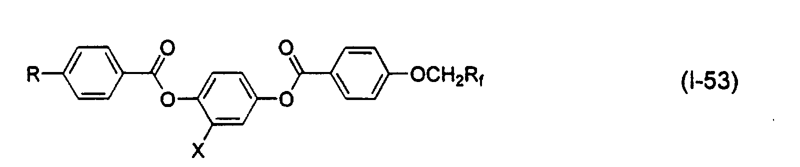 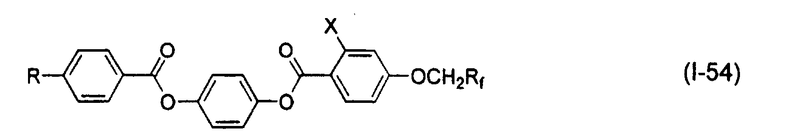 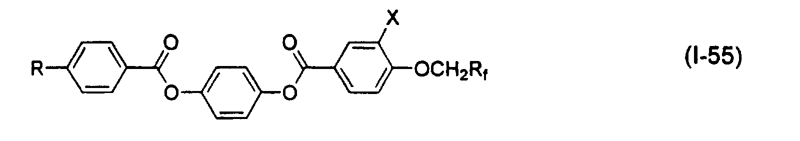 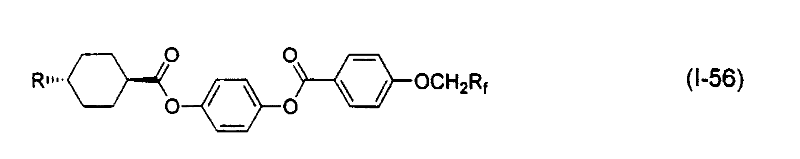 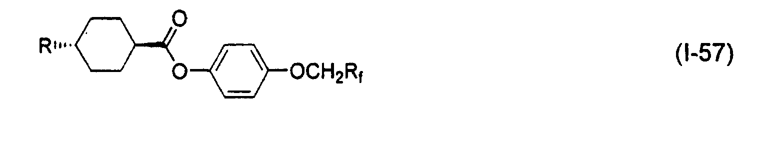 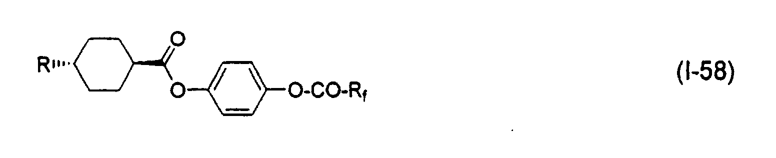 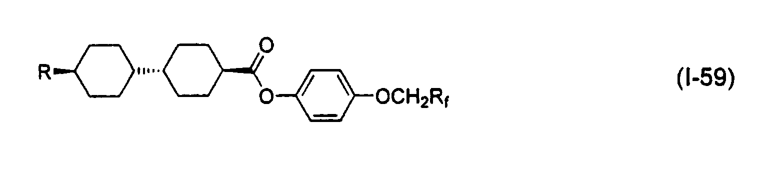 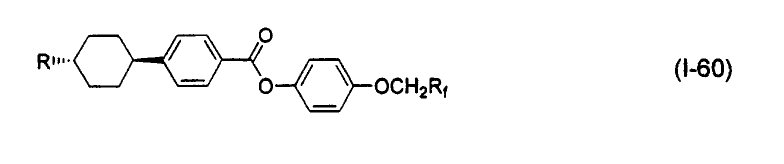 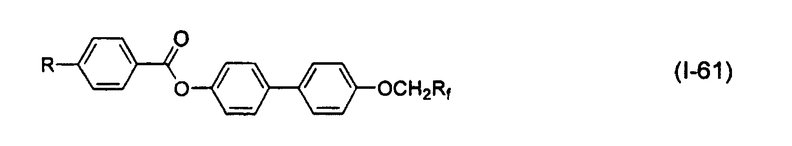 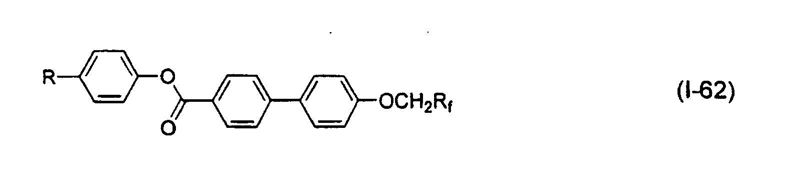 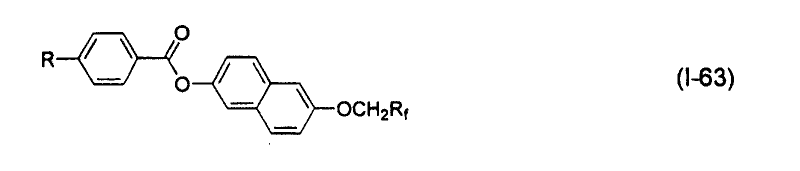 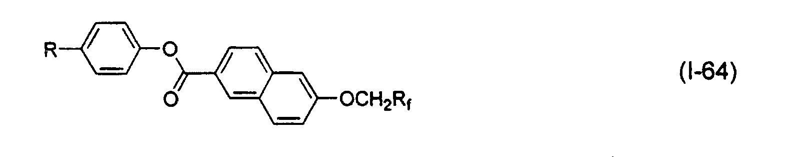 wobei X gleich F, Cl oder CN ist und R, R_{f} die für die Formel (I) angegebenen Bedeutungen und Bevorzugungen haben.

Die erfindungsgemäß verwendeten FLC-Mischungen bestehen aus mindestens 2, vorzugsweise 3 bis 30 besonders bevorzugt 4 bis 20 Komponenten.
Davon sind im allgemeinen mindestens 2, vorzugsweise 3 bis 25, besonders bevorzugt 4 bis 20 Verbindungen der Formel (I).

Die Mischungen enthalten im allgemeinen mindestens 5, vorzugsweise mindestens 20, besonders bevorzugt mindestens 50 Gew.-% an einer oder mehreren Verbindungen der Formel (I).

Arbeitsphase ist eine chiral getiltete Phase, vorzugsweise die S_{c}*-Phase. Vorzugsweise enthalten die Mischungen eine nicht optisch aktive Basismischung, vorzugsweise in einem Anteil von > 50 %, und eine oder mehrere optisch aktive Verbindungen (Dotierstoffe), die selbst flüssigkristallin sein können, aber nicht flüssigkristallin sein müssen.

Vorzugsweise beträgt im Bereich der Gebrauchstemperatur, der "layer leaning" Winkel, d.h. der Winkel zwischen smektischer Schichtennormale und der inneren Glas- bzw. Kunststoffoberfläche der Trägerplatten weniger als die Hälfte des Tiltwinkels der ferroelektrischen Flüssigkristallphase.

Der sogenannte "layer leaning" Winkel ist ein Maß für das Auftreten einer "chevron", d.h. geknickten Lagenstruktur. Er wird definiert als der Winkel zwischen der smektischen Schichtennormale und der inneren Substratoberfläche der Trägerplatten des Displays. Bei einem "layer leaning" Winkel von 0°C liegt eine "bookshelf" Anordnung vor, die eine sehr hohe Helligkeit und einen sehr hohen Kontrast ermöglicht. Darüber hinaus entstehen keine störenden "zig-zag" Defektlinien in der Flüssigkristallschicht. Je kleiner der "layer leaning" Winkel in der hier gebrauchten Definition, desto mehr stehen die smektischen Lagen senkrecht zur Trägerplatte und desto geringer ist die Ausbildung eines "chevrons". Ist der "layer leaning" Winkel jedoch gleich dem Tiltwinkel der smektischen Phase, so bildet sich ein Knick, also ein "chevron" maximaler Ausprägung bei minimaler Helligkeit und maximaler Störung durch "zig-zag" Defektlinien aus.

Vorzugsweise bestehen die Mischungen zu mehr als 75 Gew.-% aus Verbindungen der Formel (I).
Weitere Komponenten sind vorzugsweise mesogene, insbesondere smektogene und/oder netamogene Verbindungen, besonders bevorzugt mit thermodynamisch stabilen, smektischen und/oder nematischen und/oder cholesterischen Phasen.

Ganz besonders bevorzugt als solche weiteren Komponenten sind Verbindungen der Formel (II)

R¹(-A⁵-M⁴)_{d}(-A⁶-M⁵)ₑ(-A⁷-M⁶)_{g}-A⁸-R² (II)

in der
R¹, R² gleich oder verschieden unabhängig voneinander die gleichen Bedeutungen und Bevorzugungen wie R in Formel (I) haben, mit der Maßgabe, daß höchstens einer der Reste R¹, R² Wasserstoff, -F, -Cl, -CF₃, -OCF₃ oder -CN sein kann, und
M⁴, M⁵, M⁶, A⁵, A⁶, A⁷, A⁸, d, e, g gleich oder verschieden unabhängig voneinander die gleichen Bedeutungen und Bevorzugungen wie respektive M¹, M², M³, A¹, A², A³, A⁴, a, b, c in Formel (I) haben.

Dazu gehören z. B.:
- Derivate des Phenylpyrimidins, wie beispielsweise in WO 86/06401, US-4 874 542 beschrieben,
- metasubstituierte Sechsringaromaten, wie beispielsweise in EP-A 0 578 054 beschrieben,
- Siliziumverbindungen, wie beispielsweise in EP-A 0 355 008 beschrieben,
- mesogene Verbindungen mit nur einer Seitenkette, wie beispielsweise in EP-A 0 541 081 beschrieben,
- Hydrochinonderivate, wie beispielsweise in EP-A 0 603 786 beschrieben,
- Phenylbenzoate und Biphenylbenzoate, wie beispielsweise bei P. Keller, Ferroelectrics 1984, 58, 3; Liq. Cryst. 1987, 2, 63; Liq. Cryst. 1989, 5, 153 und J. W. Goodby et al., Liquid Crystals and Ordered Fluids, Bd. 4, New York 1984 beschrieben,
- Thiadiazole, wie beispielsweise in EP-A 0 309 514 beschrieben,
- Biphenyle wie beispielsweise in EP 207.712 oder Adv. Liq. Cryst. Res. Appl. (Ed. Bata, L.) 3 (1980) beschrieben,
- Phenylpyridine wie beispielsweise in Ferroelectrics 1996, 180, 269 oder Liq. Cryst. 1993, 14, 1169 beschrieben,
- Benzanilide wie beispielsweise in Liq. Cryst. 1987, 2, 757 oder Ferroelectrics 1984, 58, 81 beschrieben,
- Terphenyle wie beispielsweise in Mol. Cryst. Liq. Cryst. 1991, 195, 221; PCT-WO 89/02.425 oder Ferroelectrics 1991, 114, 207 beschrieben,
- 4-Cyanocyclohexyle wie beispielsweise in Freiburger Arbeitstagung Fluessigkristalle 1986, 16, V8 beschrieben,
- 5-Alkylthiophencarbonsäureester wie beispielsweise in Butcher, J.L., Dissertation Nottingham 1991 beschrieben und
- 1,2-Diphenylethane wie beispielsweise in Liq. Cryst. 1991, 9, 253 beschrieben,

Als chirale, nicht racemische Dotierstoffe beispielsweise:
- optisch aktive Phenylbenzoate, wie beispielsweise bei P. Keller, Ferroelectrics 1984, 58, 3 und J. W. Goodby et al., Liquid Crystals and Ordered Fluids, Bd. 4, New York 1984 beschrieben,
- optisch aktive Oxiranether, wie beispielsweise in EP-A 0 263 437 und WO-A 93/13093 beschrieben,
- optisch aktive Oxiranester, wie beispielsweise in EP-A 0 292 954 beschrieben,
- optisch aktive Dioxolanether, wie beispielsweise in EP-A 0 351 746 beschrieben,
- optisch aktive Dioxolanester, wie beispielsweise in EP-A 0 361 272 beschrieben,
- optisch aktive Tetrahydrofuran-2-carbonsäureester, wie beispielsweise in EP-A 0 355 561 beschrieben,
- optisch aktive 2-Fluoralkylether, wie beispielsweise in EP-A 0 237 007, EP-B 428 720 und US-5,051,506 beschrieben und
- optisch aktive α-Halogencarbonsäureester, wie beispielsweise in US 4,855,429 beschrieben.

Besonders bevorzugte weitere Komponenten der Formel (I) sind solche der Gruppen A bis M:
A. Phenylpyrimidinderivate der Formel (III),

   R¹-A¹-A²-R² (III)

   worin
   - R¹ und R²: jeweils Alkyl mit 1-15 C-Atomen, worin auch eine oder zwei nicht benachbarte CH₂-Gruppen durch -O-, -S-, -CO-, -O-CO-, -CO-O-, -O-CO-O-, -CO-S-, S-CO-, -CHHalogen, -CHCN- und/oder -CH=CHersetzt sein können,
   - A¹: 1,4-Phenylen, trans-1,4-Cyclohexylen oder eine Einfachbindung, und
   - A²: 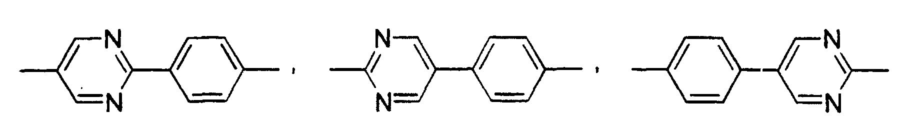 oder 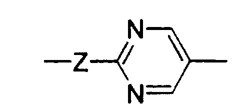 bedeutet, wobei
   - Z: -O-CO-, -CO-O-, -S-CO-, -CO-S-, -CH₂O-, -OCH₂- oder -CH₂CH₂bedeutet.
B. Verbindungen mit nur einer Seitenkette der Formel (IV),

   R¹(-A¹)ₐ(-M¹)_{b}(-A²)_{c}(-M²)_{d}(-A³)ₑ(-M³)_{f}(-A⁴)-H (IV)

   worin bedeuten:
   - R¹: ein geradkettiger oder verzweigter Alkylrest mit 1 bis 22 bzw. 3 bis 22 C-Atomen, wobei auch eine oder zwei nicht benachbarte und nicht terminale CH₂- Gruppen durch -O-, -CO-, -CO-O-, -O-CO-, -O-CO-Ooder -Si(CH₃)₂- ersetzt sein können,
   - A¹, A², A³, A⁴: gleich oder verschieden
   1,4-Phenylen, wobei ein oder zwei H-Atome durch F oder CN ersetzt sein können, Pyridin-2,5-diyl, wobei ein oder zwei H-Atome durch F ersetzt sein können, Pyrimidin-2,5-diyl, wobei ein oder zwei H-Atome durch F ersetzt sein können, trans-1,4-Cyclohexylen, (1,3,4)-Thiadiazol-2,5-diyl oder Naphthalin-2,6-diyl,
   - M¹, M², M³: gleich oder verschieden
   -CO-O-, -O-CO-, -CH₂-O-, -O-CH₂- oder -CH₂-CH₂-,
   - a, b, c, d, e, f: null oder eins,
   unter der Bedingung, daß die Summe aus a+c+e 0, 1, 2 oder 3 ist.
C. Metasubstituierte Verbindungen der Formel (V), 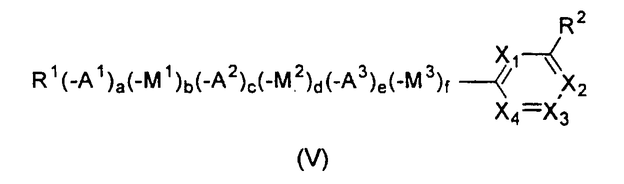 worin bedeuten:
   - R¹, R²: gleich oder verschieden
   ein geradkettiger oder verzweigter Alkylrest mit 1 bis 22 bzw. 3 bis 22 C-Atomen, wobei auch eine oder zwei nicht benachbarte und nicht terminale CH₂-Gruppen durch -O-, -CO-, -CO-O-, -O-CO-, -O-CO-Ooder -Si(CH₃)₂- ersetzt sein können,
   - A¹, A², A³: gleich oder verschieden
   1,4-Phenylen, wobei ein oder zwei H-Atome durch F ersetzt sein können, Pyridin-2,5-diyl, wobei ein oder zwei H-Atome durch F ersetzt sein können, Pyrimidin-2,5-diyl, wobei ein oder zwei H-Atome durch F ersetzt sein können, trans-1,4-Cyclohexylen, wobei ein oder zwei H-Atome durch -CN und/oder -CH₃ ersetzt sein können, (1,3,4)-Thiadiazol-2,5-diyl,
   und A¹ auch 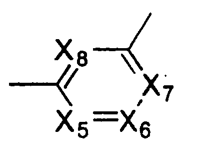
   - M¹, M², M³: gleich oder verschieden
   -O-, -CO-O-, -O-CO-, -CH₂-O-, -O-CH₂- oder -CH₂-CH₂-;
   - X¹, X², X³, X⁴, X⁵, X⁶, X⁷, X⁸: CH oder N, wobei die Zahl der N-Atome in einem Sechsring 0,1 oder 2 beträgt,
   - a, b, c, d, e, f: null oder eins,
   unter der Bedingung, daß die Summe aus a + c + e 0, 1, 2 oder 3 ist.
D. Siliziumverbindungen der Formel (VI),

   R¹(-A¹)ᵢ(-M¹)ₖ(-A²)ₗ(-M²)ₘ(-A³)ₙ-R² (VI)

   worin bedeuten:
   - R¹: ein geradkettiger oder verzweigter Alkylrest mit 1 bis 22 bzw. 3 bis 22 C- Atomen, wobei auch eine oder zwei nicht benachbarte und nicht terminale CH₂-Gruppen durch -O-, -CO-, -CO-O-, -O-CO- oder -O-CO-O- ersetzt sein können,
   - R²: ein geradkettiger oder verzweigter Alkylrest mit 1 bis 22 bzw. 3 bis 22 C-Atomen, wobei auch eine oder zwei nicht benachbarte und nicht terminale CH₂-Gruppen durch -O-, -CO-, -CO-O-, -O-CO- oder -O-CO-O- ersetzt sein können, mit der Maßgabe, daß eine nicht an Sauerstoff gebundene CH₂-Gruppe durch -Si(CH₃)₂- ersetzt ist,
   - A¹, A², A³: gleich oder verschieden
   1,4-Phenylen, wobei ein oder zwei H-Atome durch F ersetzt sein können, trans-1,4-Cyclohexylen, Pyridin-2,5-diyl, wobei ein oder zwei H-Atome durch F ersetzt sein können, Pyrimidin-2,5-diyl, wobei ein oder zwei H-Atome durch F ersetzt sein können oder (1,3,4)-Thiadiazol-2,5-diyl,
   - M¹, M²: gleich oder verschieden -CO-O-, -O-CO-, -CH₂-O-, -O-CH₂-,
   - i, k, l, m, n: null oder 1, mit der Maßgabe, daß i + I + n gleich 2 oder 3 ist.
E. Hydrochinonderivate der Formel (VII), 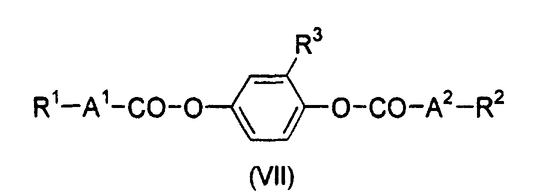 worin bedeuten:
   - R¹, R²: gleich oder verschieden
   ein geradkettiger oder verzweigter Alkylrest mit 1 bzw. 3 bis 16, vorzugsweise 1 bzw. 3 bis 10 C-Atomen, wobei auch eine oder zwei nicht benachbarte und nicht terminale CH₂-Gruppen durch -O-, -CO-, -O-CO-, -CO-O-, -O-CO-O-, vorzugsweise -O-, -O-CO-, -CO-O- ersetzt sein können,
   - R³: -CH₃, -CF₃ oder -C₂H₅, vorzugsweise -CH₃ oder -CF₃,
   - A¹, A²: gleich oder verschieden
   1,4-Phenylen, trans-1,4-Cyclohexylen, vorzugsweise 1,4-Phenylen.
F. Pyridylpyrimidine der Formel (VIII), 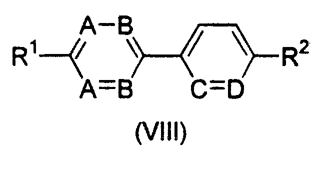 worin bedeuten:
   - A: gleich N und B gleich CH oder A gleich CH und B gleich N, C gleich N und D gleich CH oder C gleich CH und D gleich N, wobei eine oder zwei CH-Gruppen durch CF-Gruppen ersezt sein können,
   - R¹, R²: gleich oder verschieden,
   ein geradkettiger oder verzweigter Alkylrest mit 1 bis 22 bzw. 3 bis 22 C-Atomen, wobei auch eine oder zwei nicht benachbarte und nicht terminale CH₂-Gruppen durch -O-, -CO-, -CO-O-, -O-CO- oder -O-CO-O- ersetzt sein können.
G. Phenylbenzoate der Formel (IX),

   R¹(-A)ₐ(-M¹)_{b}(-A)_{c}(-M²)_{d}(-A)ₑ-R² (IX)

   wobei bedeuten:
   - R¹, R²: gleich oder verschieden
   ein geradkettiger oder verzweigter Alkylrest mit 1 bis 22 bzw. 3 bis 22 C-Atomen, wobei auch eine oder zwei nicht benachbarte und nicht terminale CH₂-Gruppen durch -O-, -CO-, -CO-O-, -O-CO- oder -O-CO-O- ersetzt sein können,
   - A: gleich 1,4-Phenylen,
   - M¹, M²: gleich oder verschieden -CO-O-, -O-CO-,
   - a, b, c, d, e: null oder eins,
   unter der Bedingung, daß a + c + e = 2 oder 3 und b + d = 1 oder 2 ist.
H. Optisch aktive Phenylbenzoate der Formel (X),

   R¹(-A)ₐ(-M¹)_{b}(-A)_{c}(-M²)_{d}(-A)ₑ-R² (X)

   wobei bedeuten:
   - R¹, R²: gleich oder verschieden
   ein geradkettiger oder verzweigter Alkylrest mit 1 bis 22 bzw. 3 bis 22 C-Atomen, wobei auch eine oder zwei nicht benachbarte und nicht terminale CH₂-Gruppen durch -O-, -CO-, -CO-O-, -O-CO- oder -O-CO-O- ersetzt sein können, und worin wenigstens einer der Reste R¹, R² eine verzweigte, optisch aktive Alkylgruppe ist,
   - A: gleich 1,4-Phenylen,
   - M¹, M²: gleich oder verschieden
   -CO-O-, -O-CO- oder eine Einfachbindung,
   - a, b, c, d, e: null oder eins,
   unter der Bedingung, daß a + c + e = 2 oder 3 und b + d = 1 oder 2 ist.
I. Optisch aktive Oxiranether der Formel (XI) 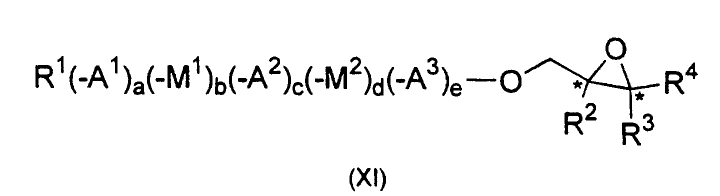 wobei die Symbole und Indizes folgende Bedeutung haben:
   * ein chirales Zentrum

   - R¹: ein geradkettiger oder verzweigter Alkylrest mit 1 bis 22 bzw. 3 bis 22 C-Atomen, wobei auch eine oder zwei nicht benachbarte und nicht terminale CH₂-Gruppen durch -O-, -CO-, -CO-O-, -O-CO-, -O-CO-Ooder -Si(CH₃)₂- ersetzt sein können,
   oder die nachfolgende, optisch aktive Gruppe, 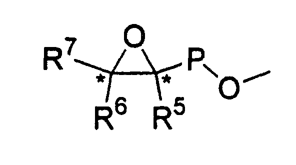
   - R², R³, R⁴, R⁵, R⁶, R⁷: gleich oder verschieden
   Wasserstoff oder ein geradkettiger oder verzweigter Alkylrest mit 1 bis 16 C-Atomen,
   - P: -CH₂- oder -CO-,
   - A¹, A², A³: sind gleich oder verschieden
   1,4-Phenylen, wobei ein oder zwei H-Atome durch F ersetzt sein können, Pyridin-2,5-diyl, wobei ein oder zwei H-Atome jeweils durch F ersetzt sein können, Pyrimidin-2,5-diyl, wobei ein oder zwei H-Atome durch F ersetzt sein können, trans-1,4-Cyclohexylen, wobei ein oder zwei H-Atome durch -CN und/oder -CH₃ ersetzt sein können, (1,3,4)-Thiadiazol-2,5-diyl,
   - M¹, M²: gleich oder verschieden
   -CO-O-, -O-CO-, -CH₂-O-, -O-CH₂-, -CH₂-CH₂-,
   - a, b, c, d, e: null oder eins.

   Die asymmetrischen C-Atome des Oxiranrings oder der Oxiranringe können gleich oder verschieden R oder S konfiguriert sein.
J. Optisch aktive Oxiranester der Formel (XII), 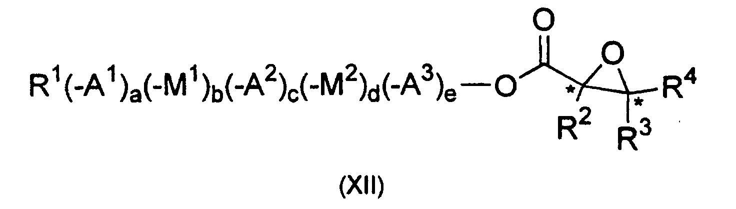 wobei die Symbole und Indizes folgende Bedeutung haben:
   * ein chirales Zentrum

   - R¹: ein geradkettiger oder verzweigter Alkylrest mit 1 bis 22 bzw. 3 bis 22 C-Atomen, wobei auch eine oder zwei nicht benachbarte und nicht terminale CH₂-Gruppen durch -O-, -CO-, -CO-O-, -O-CO-, -O-CO-O- oder -Si(CH₃)₂- ersetzt sein können,
   - R², R³, R⁴: gleich oder verschieden
   Wasserstoff oder ein geradkettiger oder verzweigter Alkylrest mit 1 bis 16 C-Atomen,
   - A¹, A², A³: gleich oder verschieden
   1,4-Phenylen, wobei ein oder zwei H-Atome durch F ersetzt sein können, Pyridin-2,5-diyl, wobei ein oder zwei H-Atome jeweils durch F ersetzt sein können, Pyrimidin-2,5-diyl, wobei ein oder zwei H-Atome durch F ersetzt sein können, trans-1,4-Cyclohexylen, wobei ein oder zwei H-Atome durch -CN und/oder -CH₃ ersetzt sein können, (1,3,4)-Thiadiazol-2,5-diyl,
   - M¹, M²: gleich oder verschieden
   -CO-O-, -O-CO-, -CH₂-O-, -O-CH₂-, -CH₂-CH₂-,
   - a, b, c, d, e: null oder eins.

   Die asymmetrischen C-Atome des Oxiranrings können gleich oder verschieden R oder S konfiguriert sein.
K. Optisch aktive Dioxolanether der Formel (XIII), 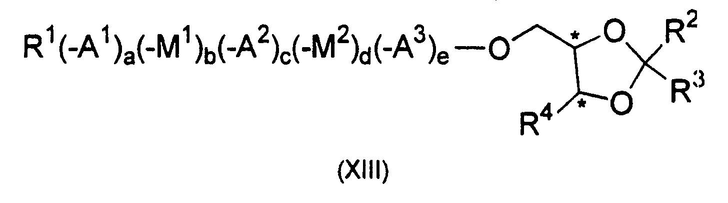 wobei die Symbole und Indizes folgende Bedeutung haben:
   * ein chirales Zentrum

   - R¹: ein geradkettiger oder verzweigter Alkylrest mit 1 bis 22 bzw. 3 bis 22 C-Atomen, wobei auch eine oder zwei nicht benachbarte und nicht terminale CH₂-Gruppen durch -O-, -CO-, -CO-O-, -O-CO-, -O-CO-O- oder -Si(CH₃)₂- ersetzt sein können,
   - R², R³, R⁴: gleich oder verschieden
   Wasserstoff, ein geradkettiger oder verzweigter Alkylrest mit 1 bis 16 bzw. 3 bis 10 C-Atomen oder ein Alkenylrest mit 2 bis 16 C-Atomen, wobei R² und R³ zusammen auch -(CH₂)₅- sein können,
   - A¹, A², A³: gleich oder verschieden
   1,4-Phenylen, wobei ein oder zwei H-Atome durch F ersetzt sein können, Pyridin-2,5-diyl, wobei ein oder zwei H-Atome durch F ersetzt sein können, Pyrimidin-2,5-diyl, wobei ein oder zwei H-Atome durch F ersetzt sein können, trans-1,4-Cyclohexylen, wobei ein oder zwei H-Atome durch -CN und/oder -CH₃ ersetzt sein können, (1,3,4)-Thiadiazol-2,5-diyl,
   - M¹, M²: gleich oder verschieden
   -CO-O-, -O-CO-, -CH₂-O-, -O-CH₂-, -CH₂-CH₂-,
   - a, b, c, d, e: null oder eins.

   Asymmetrische C-Atome des Dioxolanrings können, gleich oder verschieden, R oder S konfiguriert sein.
L. Optisch aktive Dioxolanester der Formel (XIV), 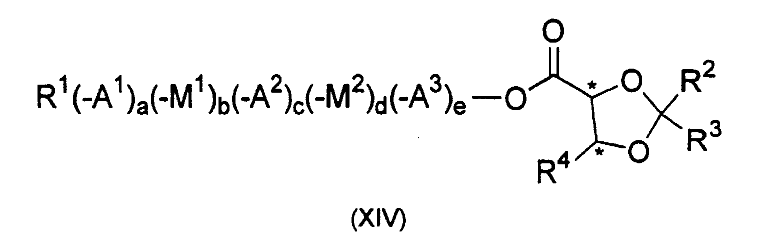 wobei die Symbole und Indizes folgende Bedeutung haben:
   * ein chirales Zentrum

   - R¹: ein geradkettiger oder verzweigter Alkyrest mit 1 bis 16 bzw. 3 bis 16 C-Atomen, wobei eine oder mehrere nicht benachbarte und nicht terminale CH₂ Gruppen durch -O-, -CO-, -O-CO- oder -CO-O- ersetzt sein können,
   - R², R³, R⁴: gleich oder verschieden
   Wasserstoff oder ein Alkyl- oder Alkenylrest mit 1 bis 10 bzw. 2 bis 10 C-Atomen, wobei R² und R³ zusammen auch -(CH₂)₅- sein können,
   - A¹, A², A³: sind gleich oder verschieden
   1,4-Phenylen, wobei ein oder zwei H-Atome durch F ersetzt sein können, Pyridin-2,5-diyl, wobei ein oder zwei H-Atome durch F ersetzt sein können, Pyrimidin-2,5-diyl, wobei ein oder zwei H-Atome durch F ersetzt sein können, trans-1,4-Cyclohexylen, wobei ein oder zwei H-Atome durch -CN und/oder -CH₃ ersetzt sein können, (1,3,4)-Thiadiazol-2,5-diyl,
   - M¹, M²: gleich oder verschieden
   -CO-O-, -O-CO-, -CH₂-O-, -O-CH₂-, -CH₂-CH₂-,
   - a, b, c, d, e: null oder eins.

   Asymmetrische C-Atome des Dioxolanrings können, gleich oder verschieden, R oder S konfiguriert sein.
M. Makrocyclische Verbindungen der Formel (XV), 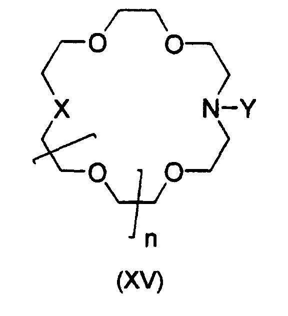 mit
   - n:: 0, 1
   - Y:: -CO-(t-Butyl), -CO-(Adamantyl), -CO-Alkyl
   - X:: -O-, -N(Y)-.

Die Herstellung der Flüssigkristallkomponenten der Formel (I) bis (XV) erfolgt nach an sich bekannten, dem Fachmann geläufigen Methoden, wie sie beispielsweise in Houben Weyl, Methoden der Organischen Chemie, Georg Thieme Verlag, Stuttgart oder auch den zitierten Schriften beschrieben werden.

Insbesondere wird die Herstellung von Verbindungen der Formel (I) z. B. beschrieben in E. P. Janulis et al., Ferroelectrics 1988, 85, 375-384; H. T. Nguyen et al., Liq. Cryst. 1991, 10, 389; S. Misaki et al., Mol. Cryst. Liq. Cryst. 1981, 66, 123-132; L. M. Yagupolski et al., Mol. Cryst. Liq. Cryst. 1980, 56 (Letters), 209-215; A. V. Ivashchenko et al., Mol. Cryst. Liq. Cryst. 1981, 67, 235-240; H. Liu, H. Nohira, Liq. Cryst. 1996, 20, 581-586; ibid. 1997, 22, 217-222; EP-B 255 236; WO 91/00897; WO 93/22396; WO 96/15092.

Die Herstellung der Mischung kann nach an sich bekannten Methoden erfolgen.

Das erfindungsgemäße Display enthält Polarisatoren, Elektroden, z. B. aus Indium-Zinn-Oxid, Substrate aus Kunststoff oder dünnem Glas, die mit einer Orientierungsschicht und möglicherweise weiteren Funktionsschichten (Passivierungs-, Diffusionssperr-, Isolations-, Antireflex- usw. Schichten) versehen sind, einer Flüssigkristallschicht und aktiven Dünnschichtelementen. Letztere umfaßt die möglichen Typen aSi-TFT, pSi-TFT, Dioden, Metall-Insulator-Metall (MIM) Elemente. Das erfindungsgemäße Display kann auch vom Typ in-plane switching-LCD, AM-ECB oder ein ähnlich arbeitendes active matrix Flüssigkristalldisplay sein. (siehe z. B. C. Prince, Seminar Lecture Notes, Volume I, p. M-3/3-M-3/22, SID International Symposium 1997, Boston, USA; B. B.Bahadur, Liquid Crystals Application and Uses, Vol. 1, pp. 410, World Scientific Publishing, 1990; E. Lüder, Recent Progress of AMLCD's, Proceedings of the 15^{th} international display research conference, 1995, p.9 -p.12).

Darüber hinaus können solche Displays vorteilhaft Elektrodenabstände von mehr als 1,5 µm, insbesondere von mehr als 1,8 µm aufweisen und lassen sich dennoch bei niedrigen Spannungen unterhalb von 30 Volt schalten. Der im Vergleich zu den bisher zitierten FLC -Displays große Elektrodenabstand ermöglicht eine hohe Ausbeute bei der Fertigung.

Die Ansteuerung des erfindungsgemäßen Displays kann wie in der einschlägigen Literatur ausführlich beschrieben erfolgen (siehe z.B. C. Prince, Seminar Lecture Notes, Volume I, p. M-3/3-M-3/22, SID International Symposium 1997, Boston, USA oder T. Tsukuda, siehe oben).

Entscheidend für die elektro-optischen Eigenschaften und Speichereigenschaften des Displays ist die ca. 1-3 µm dicke FLC-Schicht, deren Schichtdicke,
vorzugsweise durch Abstandshalter festgelegt wird. Diese Abstandshalter können eingemischte Teilchen, wie Kugeln, oder auch strukturierte Säulen im Displayinneren sein.

Die gesamte, üblicherweise mit einem Kleberahmen verschlossene Zelle kann elektrisch, beispielsweise durch Löten, Bonden, Pressen o.ä. kontaktiert werden.

Der elektrooptische Effekt, der vorzugsweise auf der Doppelbrechung des FLC Materials oder auf der anisotropen Absorption eines eingemischten dichroitischen Farbstoffs beruht, wird zwischen zwei gekreuzten Polarisatoren (Polarisationsfolien) bzw. einem Polarisator ('guest-host' Modus, reflektiver Modus) sichtbar.

Die Herstellung des erfindungsgemäßen FLC-Displays kann nach grundsätzlich bekannten Verfahren erfolgen, wie sie beispielsweise bei Tsukuda beschrieben sind.

Auf die in dieser Anmeldung zitierten Literaturstellen wird ausdrücklich Bezug genommen; sie sind durch Zitat Bestandteil der Beschreibung.

Die Erfindung wird durch die nachfolgenden Beispiele weiter erläutert, ohne sie dadurch beschränken zu wollen.

### Beispiele

### Beispiel 1:

### Synthese von 2-{4-[2,2-Difluor-2-(1,1,2,2-tetrafluor-2-nonafluorbutyloxy-ethoxy)-ethoxy]-phenyl}-5-octyloxypyrimidin

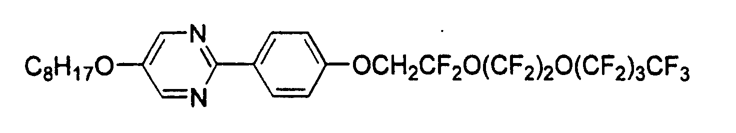

Eine Suspension von Natriumhydrid in trockenem Dimethylformamid wird vorgelegt und bei 0°C eine Lösung von 2-{4-[2,2-Difluor-2-(1,1,2,2-tetrafluor-2-nonafluorbutyloxy-ethoxy)-ethoxy]-phenyl}-pyrimidin-5-ol (erhalten durch Williamson-Veretherung von 5-Benzyloxy-2-(4-hydroxyphenyl)-pyrimidin mit Toluol-4-sulfonsäure-2,2-difluor-2-(1,1,2,2-tetrafluor-2-nonafluorbutyloxy-ethoxy)-ethylester und anschließender Hydrierung) im gleichen Lösemittel zugetropft. Man rührt bei Raumtemperatur, bis die Gasentwicklung beendet ist und tropft anschließend die äquivalente Menge 1-Bromoctan zu. Es wird 4-6 h auf ca. 60°C erwärmt. Nach dem Abkühlen gibt man die Reaktionsmischung auf Eiswasser und extrahiert mehrmals mit Dichlormethan. Die vereinigten org. Extrakte werden mit ges.
Natriumchloridlösung gewaschen und mit Natriumsulfat getrocknet. Das Lösemittel wird im Vakuum entfernt und das Rohprodukt säulenchromatographisch an Kieselgel und durch Umkristallisation aus Ethanol gereinigt.
Phasenübergänge (in °C): X 45 S_{C} 81 S_{A} 94 l.

### Beispiel 2: Mischungsbeispiel

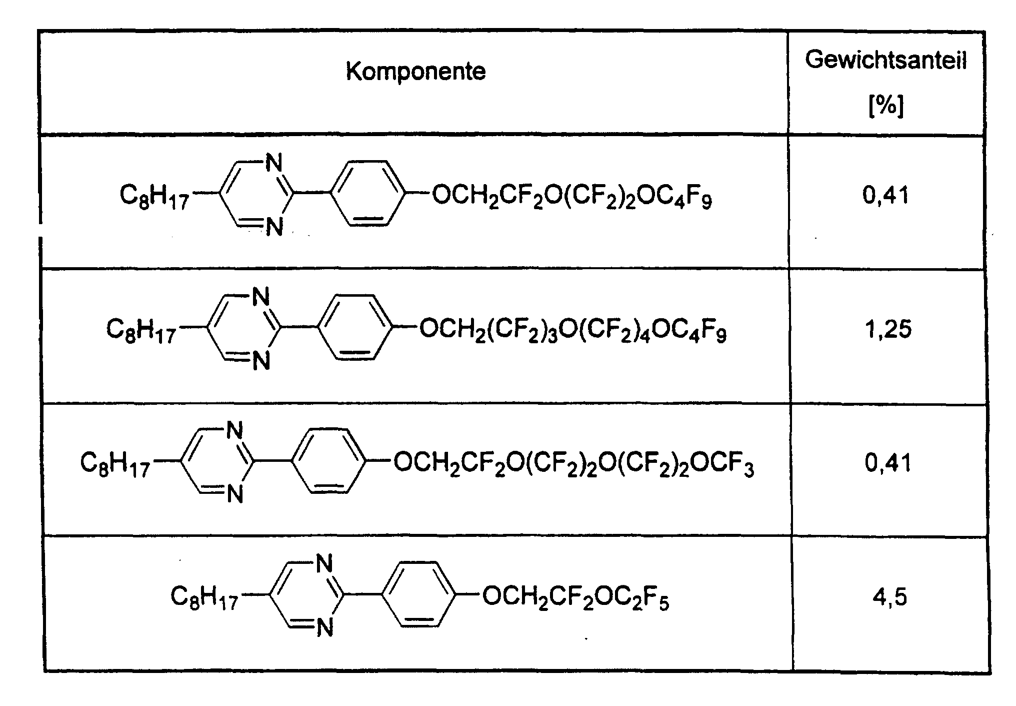

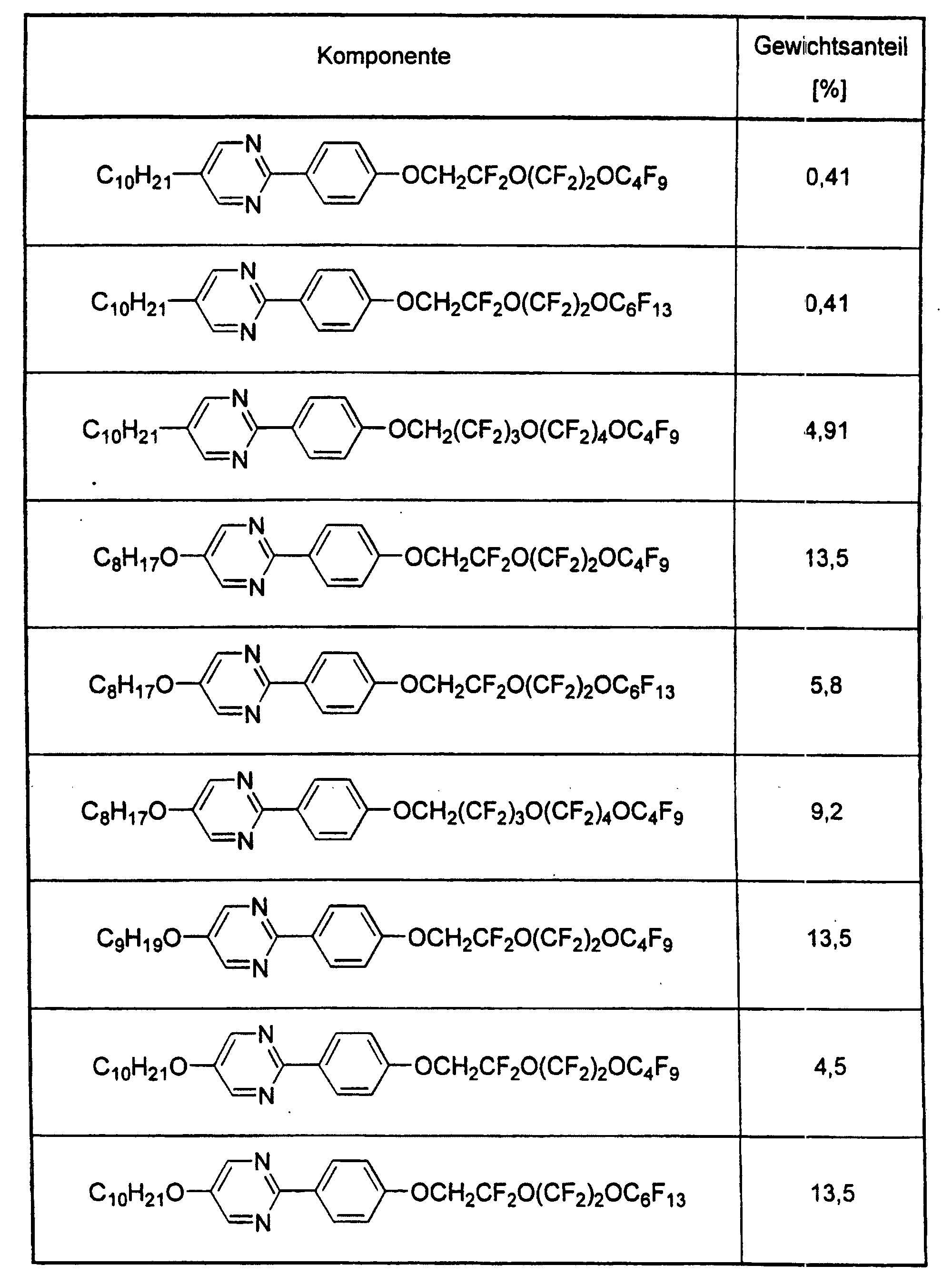

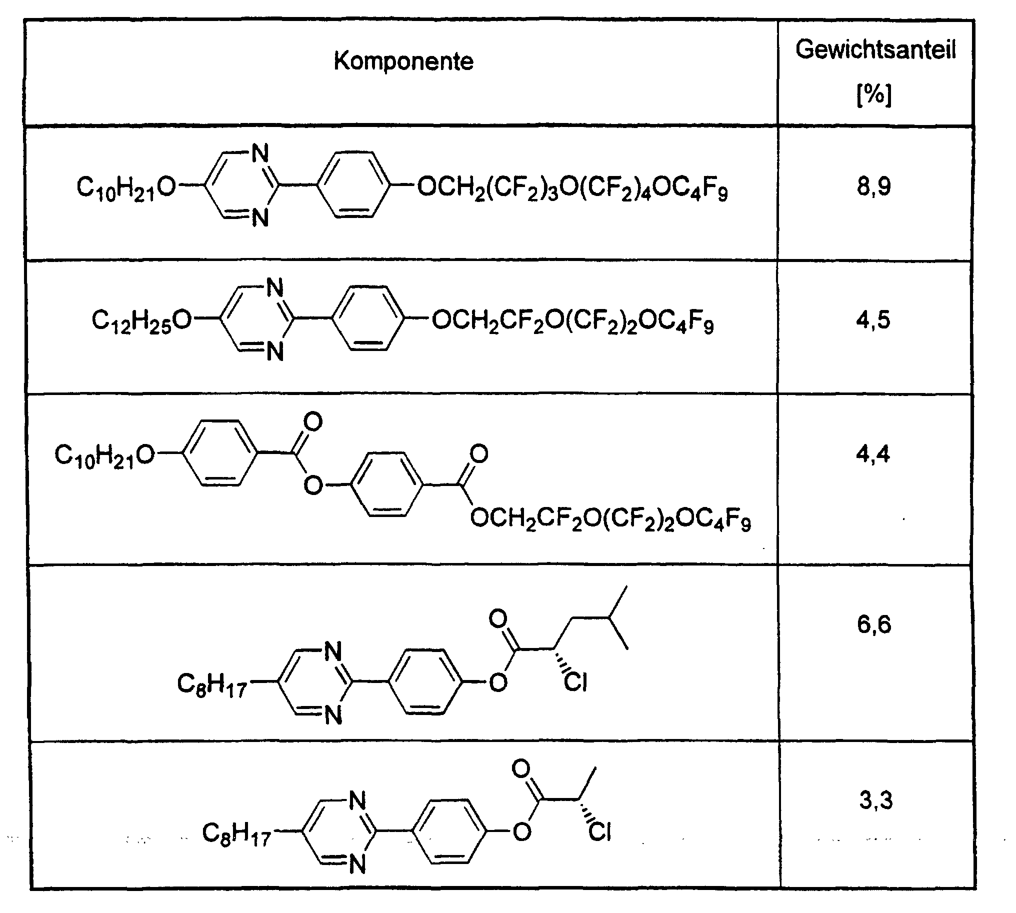

## Patentansprüche

1. Ferroelektrische Aktivmatrix-Flüssigkristallanzeige, enthaltend einen ferroelektrischen Flüssigkristall, der eine oder mehrere mesogene Verbindungen mit teil- oder perfluorierter Seitenkette der Formel (I) enthält,
R(-A¹-M1)ₐ-(-A²⁻M²)_{b}(-A³-M³)_{c}-B-R_{f} (I)
in der die Symbole und Indizes folgende Bedeutungen haben:
R ist
a) Wasserstoff, -F, -Cl, -CF₃, -OCF₃ oder -CN,
b) ein geradkettiger oder verzweigter Alkylrest (mit oder ohne asymmetrisches C-Atom) mit 1 bis 20 C-Atomen, wobei
b1) eine oder mehrere nicht benachbarte und nicht terminale CH₂-Gruppen durch -O-, -S-, -CO-, -CO-O-, -O-CO-, -O-CO-O- oder -Si(CH₃)₂- ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F, CN und/oder Cl ersetzt sein können und/oder
b4) die terminale CH₃-Gruppe durch eine der folgenden chiralen Gruppen (optisch aktiv oder racemisch) ersetzt sein kann: 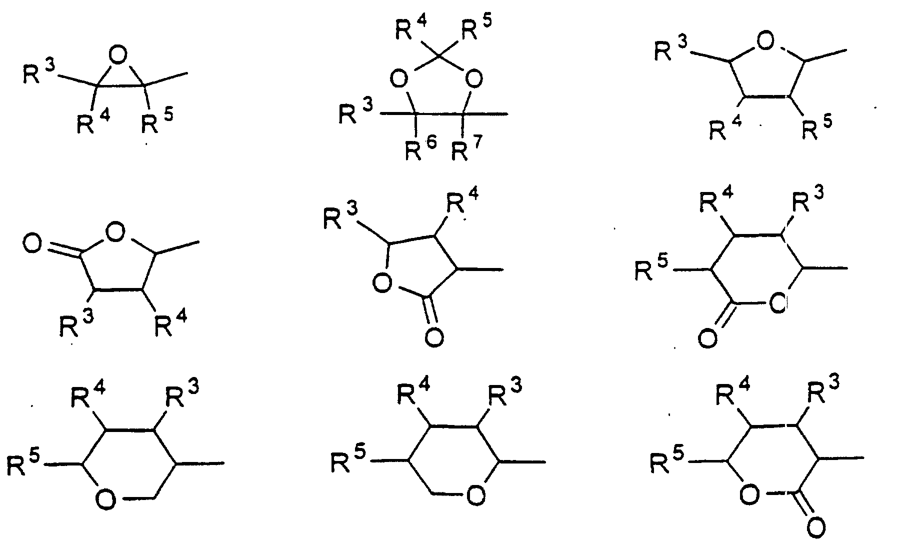 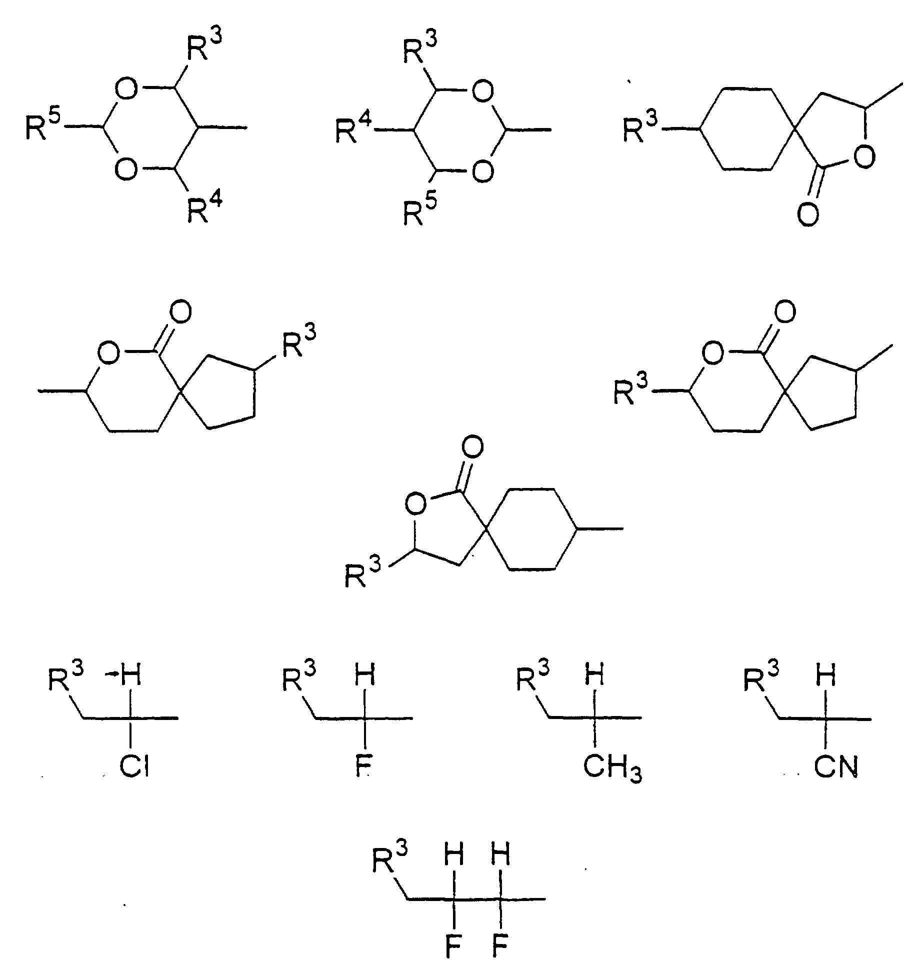
c) B-R_{f}.
R³, R⁴, R⁵, R⁶, R⁷ sind gleich oder verschieden
a) Wasserstoff
b) ein geradkettiger oder verzweigter Alkylrest (mit oder ohne asymmetrisches Kohlenstoffatom) mit 1 bis 16 C-Atomen, wobei
b1) eine oder mehrere nicht benachbarte und nicht terminale CH₂-Gruppen durch -O- ersetzt sein können und/oder
b2) eine oder zwei CH₂-Gruppen durch -CH=CH- ersetzt sein können,
c) R⁴ und R⁵ zusammen auch -(CH₂)₄- oder -(CH₂)₅-, wenn sie an ein Oxiran-, Dioxolan-, Tetrahydrofuran-, Tetrahydropyran-, Butyrolacton- oder Valerolacton-System gebunden sind;
R_{f} ist
ein geradkettiger oder verzweigter teil- oder perfluorierter Alkylrest (mit oder ohne asymmetrisches C-Atom) mit 1 bis 20 C-Atomen, wobei
a) eine oder mehrere nicht benachbarte und nicht terminale CH₂- oder CF₂-Gruppen durch -O-, -S-, -CO-, -CO-O-, -O-CO-, -O-CO-O- oder -Si(CH₃)₂ersetzt sein können und/oder
b) eine oder mehrere CH₂- oder CF₂-Gruppen durch -CH=CH-, -C≡C-, Cyclopropan-1,2-diyl, 1,4-Phenylen, 1,4-Cyclohexylen oder 1,3-Cyclopentylen ersetzt sein können;
B ist
-O-, -S-, -(CH₂)ₙ₊₁-O-, -O-(CH₂)ₙ₊₁-, -(CH₂)ₙ₊₁-S-, -S-(CH₂)ₙ₊₁-, -CO-(CH₂)ₙ-, -CO-O-(CH₂)ₙ-, -O-CO-(CH₂)ₙ-, -CO-S-(CH₂)ₙ-, -S-CO-(CH₂)ₙ-, -CS-O-(CH₂)ₙ-, -O-CS-(CH₂)ₙ-, -SO₂-(CH₂)ₙ-, -OSO₂-(CH₂)ₙ-, -CH=CH-, -C≡C-, -(CH₂)ₙ₊₁-, -CH=N-, -N(CₖH₂ₖ₊₁)-, -(CH₂)ₙ-N(CₖH₂ₖ₊₁)-CO-, -(CH₂)ₙ-N(CₖH₂ₖ₊₁)-SO₂-, -O-[(CH₂)ₘ₊₁-O]ₗ-(CH₂)ₙ-, -[(CH₂)ₘ₊₁-O]ₗ-(CH₂)ₙ- oder eine Einfachbindung;
m, n sind gleich oder verschieden unabhängig voneinander eine ganze Zahl von 0 bis 15, k ist eine ganze Zahl von 0 bis 4 und I ist eine ganze Zahl von 1 bis 6, mit der Maßgabe, daß m+n ≤ 15 ist;
M¹, M², M³ sind gleich oder verschieden
-CO-O-, -O-CO-, -CO-S-, -S-CO-, -CS-O-, -O-CS-, -CS-S-, -S-CS-, -CH₂-O-, -O-CH₂-, -CH₂-S-, -S-CH₂-, -CH₂-CH₂-, -CH=CH-, -C≡C-, -CH₂-CH₂-CO-O-, -O-CO-CH₂-CH₂-, -CH=N- oder eine Einfachbindung;
A¹, A², A³, A⁴ sind gleich oder verschieden
1,4-Phenylen, wobei ein oder mehrere H-Atome durch F, Cl, CH₃, C₂H₅, OCH₃, CF₃, OCF₃ und/oder CN ersetzt sein können, 1,3-Phenylen, wobei eine oder zwei CH-Gruppen durch N ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, 1,4-Cyclohexylen, wobei ein oder zwei H-Atome durch CN und/oder CH₃ und/oder F ersetzt sein können, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F, Cl und/oder CN ersetzt sein kann, 1,3-Thiazol-2,5-diyl, wobei ein H-Atom durch F, Cl und/oder CN ersetzt sein kann, Thiophen-2,4-diyl, wobei ein H-Atom durch F, Cl und/oder CN ersetzt sein kann, Thiophen-2,5-diyl, wobei ein oder zwei H-Atome durch F, Cl und/oder CN ersetzt sein können, Naphthalin-2,6-diyl, Naphtalin-1,4-diyl oder Naphtalin-1,5-diyl, wobei jeweils ein oder mehrere H-Atome durch F, Cl und/oder CN ersetzt sein können und/oder eine oder zwei CH-Gruppen durch N ersetzt sein können, Phenanthren-2,7-diyl oder 9,10-Dihydrophenanthren-2,7-diyl, wobei jeweils ein, zwei oder mehrere H-Atome durch F ersetzt sein können und/oder eine oder zwei CH-Gruppen durch N ersetzt sein können, Indan-2,5-diyl, Indan-1-on-2,5-diyl, Benzothiazol-2,6-diyl, Benzothiazol-2,5-diyl, Benzoxazol-2,6-diyl, Benzoxazol-2,5-diyl, Benzofuran-2,5-diyl, Benzofuran-2,6-diyl, 2,3-Dihydrobenzofuran-2,5-diyl, Piperazin-1,4-diyl, Piperazin-2,5-diyl, 1-Alkyl-1-silacyclohexylen-1,4-diyl oder 1,3-Dioxaborinan-2,5-diyl;
a, b, c sind null oder eins,
mit der Maßgabe, daß die Verbindung der Formel (I) nicht mehr als vier fünf- oder mehrgliedrige Ringsysteme enthalten darf.

2. Ferroelektrische Aktivmatrix-Flüssigkristallanzeige nach Anspruch 1, **dadurch gekennzeichnet, daß** die Anzeige Elektrodenabstände von mindestens 1,5 µm aufweist und bei Spannungen von ≤ 30 V geschaltet werden kann.

3. Ferroelektrische Aktivmatrix-Flüssigkristallanzeige nach Anspruch 1 oder 2, **dadurch gekennzeichnet, daß** eine Flüssigkristallmischung mit 3 bis 30 Komponenten eingesetzt wird, wovon 3 bis 25 der Komponenten Verbindungen der Formel (I) sind.

4. Verwendung eines ferroelektrischen Flüssigkristalls gemäß Anspruch 1 in Aktivmatrix-Flüssigkristallanzeigen.

## Claims

1. A ferroelectric active-matrix liquid-crystal display containing a ferroelectric liquid crystal containing one or more mesogenic compounds having a partially or perfluorinated side chain of the formula (I)
R(-A¹-M¹)ₐ(-A²-M²)_{b}-(-A³-M³)_{c}-A⁴-B-R_{f} (I)
where the symbols and indices are defined as follows:
R is
a) hydrogen, -F, -Cl, -CF₃, -OCF₃ or -CN,
b) a straight-chain or branched alkyl radical (with or without an asymmetrical carbon atom) having 1 to 20 carbon atoms, where
b1) one or more non-adjacent and non-terminal CH₂- groups may be replaced by -O-, -S-, -CO-, -CO-O-, -O-CO-, -O-CO-O- or -Si(CH₃)₂-, and/or
b2) one or more CH₂- groups may be replaced by -CH=CH-, -C≡C-, cyclopropane-1,2-diyl, 1,4-phenylene, 1,4-cyclohexylene or 1,3-cyclopentylene, and/or
b3) one or more H atoms may be replaced by F, CN and/or Cl, and/or
b4) the terminal CH₃ group may be replaced by one of the following chiral groups (optically active or racemic): 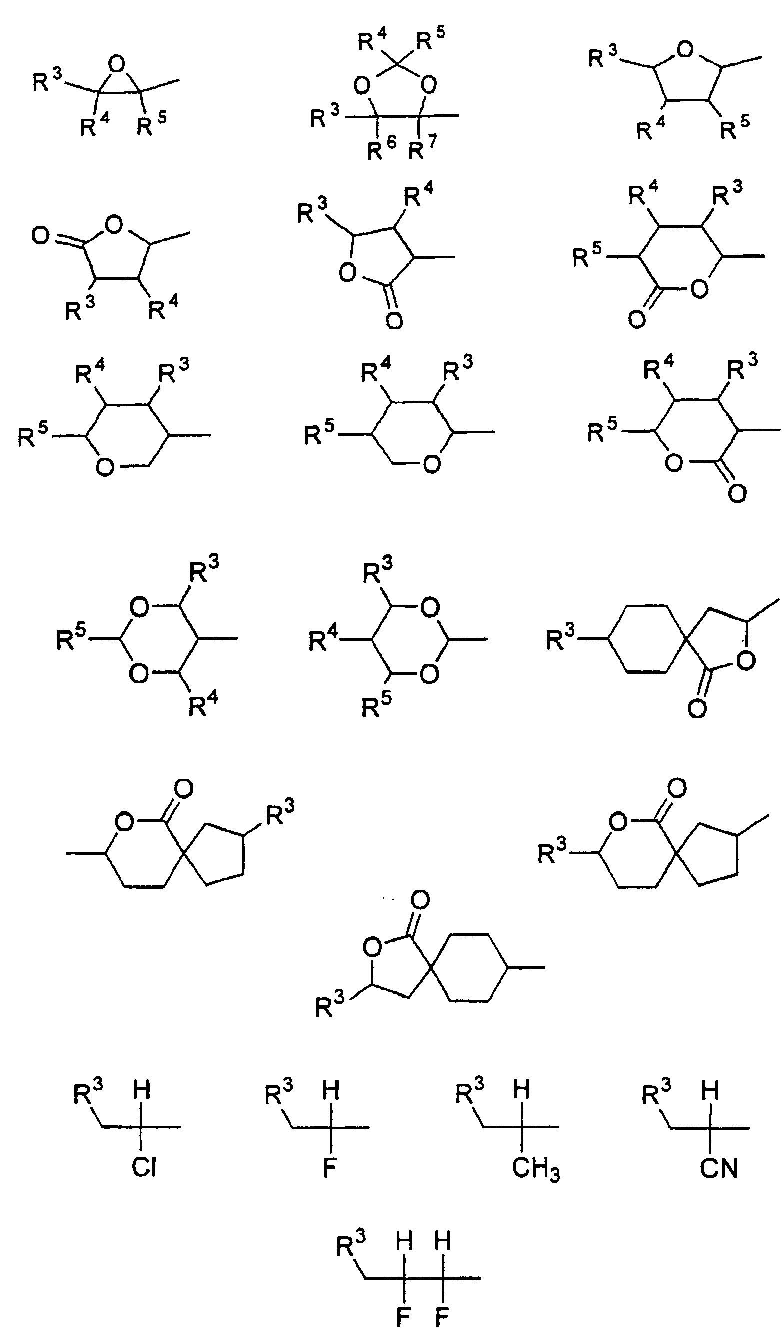
c) B-R_{f}
R³, R⁴, R⁵, R⁶ and R⁷ are identical or different and are
a) hydrogen
b) a straight-chain or branched alkyl radical (with or without an asymmetrical carbon atom) having 1 to 16 carbon atoms, where
b1) one or more non-adjacent and non-terminal CH₂- groups may be replaced by -O-, and/or
b2) one or two CH_{2⁻} groups may be replaced by -CH=CH-,
c) R⁴ and R⁵ together may alternatively be -(CH₂)₄- or -(CH₂)₅- if they are bonded to an oxirane, dioxolane, tetrahydrofuran, tetrahydropyran, butyrolactone or valerolactone system;
R_{f} is
a straight-chain or branched, partially or perfluorinated alkyl radical (with or without an asymmetrical carbon atom) having 1 to 20 carbon atoms, where
a) one or more non-adjacent and non-terminal CH₂- or CF₂-groups may be replaced by -O-, -S-, -CO-, -CO-O-, -O-CO-, -O-CO-O- or -Si(CH₃)₂-, and/or
b) one or more CH₂- or CF₂- groups may be replaced by -CH=CH-, -C≡C-, cyclopropane-1,2-diyl, 1,4-phenylene, 1,4-cyclohexylene or 1,3-cyclopentylene;
B is
-O-, -S-, -(CH₂)ₙ₊₁-O-, -O-(CH₂)ₙ₊₁-, -(CH₂)ₙ₊₁-S-, -S-(CH₂)ₙ₊₁-, -CO-(CH₂)ₙ-, -CO-O-(CH₂)ₙ-, -O-CO-(CH₂)ₙ-, -CO-S-(CH₂)ₙ-, -S-CO-(CH₂)ₙ-, -CS-O-(CH₂)ₙ-, -O-CS-(CH₂)ₙ-, -SO₂-(CH₂)ₙ-, -OSO₂-(CH₂)ₙ-, -CH=CH-, -C≡C-, -(CH₂)ₙ₊₁-, -CH=N-, -N(CₖH₂ₖ₊₁)-, -(CH₂)ₙ-N(CₖH₂ₖ₊₁)-CO-, -(CH₂)ₙ-N(CₖH₂ₖ₊₁)-SO₂-, -O-[(CH₂)ₘ₊₁-O]ₗ-(CH₂)ₙ-, -[(CH₂)ₘ₊₁-O]ₗ-(CH₂)ₙ- or a single bond;
m and n are identical or different and are, independently of one another, an integer from 0 to 15, k is an integer from 0 to 4, and I is an integer from 1 to 6, with the proviso that m + n ≤ 15;
M¹, M² and M³ are identical or different and are
-CO-O-, -O-CO-, -CO-S-, -S-CO-, -CS-O-, -O-CS-, -CS-S-, -S-CS-, -CH₂-O-, -O-CH₂-, -CH₂-S-, -S-CH₂-, -CH₂-CH₂-, -CH=CH-, -C≡C-, -CH₂-CH₂-CO-O-, -O-CO-CH₂-CH₂-, -CH=N- or a single bond;
A¹, A², A³ and A⁴ are identical or different and are
1,4-phenylene, in which one or more H atoms may be replaced by F, Cl, CH₃, C₂H₅, OCH₃, CF₃, OCF₃ and/or CN, 1,3-phenylene, in which one or two CH groups may be replaced by N, pyrazine-2,5-diyl, in which one or two H atoms may be replaced by F, Cl and/or CN, pyridazine-3,6-diyl, in which one or two H atoms may be replaced by F, Cl and/or CN, pyridine-2,5-diyl, in which one or more H atoms may be replaced by F, Cl and/or CN, pyrimidine-2,5-diyl, in which one or two H atoms may be replaced by F, Cl and/or CN, 1,4-cyclohexylene, in which one or two H atoms may be replaced by CN and/or CH₃ and/or F, 1,3,4-thiadiazole-2,5-diyl, 1,3-dioxane-2,5-diyl, 1,3-dithiane-2,5-diyl, 1,3-thiazole-2,4-diyl, in which one H atom may be replaced by F, Cl and/or CN, 1,3-thiazole-2,5-diyl, in which one H atom may be replaced by F, Cl and/or CN, thiophene-2,4-diyl, in which one H atom may be replaced by F, Cl and/or CN, thiophene-2,5-diyl, in which one or two H atoms may be replaced by F, Cl and/or CN, naphthalene-2,6-diyl, naphthalene-1,4-diyl or naphthalene-1,5-diyl, in each of which one or more H atoms may be replaced by F, Cl and/or CN and/or one or two CH groups may be replaced by N, phenanthrene-2,7-diyl or 9,10-dihydrophenanthrene-2,7-diyl, in each of which one, two or more H atoms may be replaced by F and/or one or two CH groups may be replaced by N, indane-2,5-diyl, indan-1-one-2,5-diyl, benzothiazole-2,6-diyl, benzothiazole-2,5-diyl, benzoxazole-2,6-diyl, benzoxazole-2,5-diyl, benzofuran-2,5-diyl, benzofuran-2,6-diyl, 2,3-dihydrobenzofuran-2,5-diyl, piperazine-1,4-diyl, piperazine-2,5-diyl, 1- alkyl-1-silacyclohexylene-1,4-diyl or 1,3-dioxaborinane-2,5-diyl;
a, b and c are zero or one,
with the proviso that the compound of the formula (I) cannot contain more than four five- or multimembered ring systems.

2. A ferroelectric active matrix display according to claim 1, wherein the display has electrodes spaced at least 1,5 µm and which can be switched at voltages of less or equal 30 V.

3. A ferroelectric active matrix display according to claim 1 or 2,
wherein a liquid crystal mixture of 3 to 30 components is employed of which 3 to 25 components are compounds of the formula (I).

4. The use of a ferroelectric liquid crystal according to claim 1 in active-matrix displays.

## Revendications

1. Afficheur à cristaux liquides à matrice active ferroélectrique contenant un cristal liquide ferroélectrique qui contient un ou plusieurs composés mésogènes possédant des chaînes latérales partiellement fluorées ou perfluorées de formule (I)
R(-A¹-M¹)ₐ(-A²-M²)_{b}(-A³-M³)_{c}-B-R₁ (I)
dans laquelle les symboles et les indices possèdent les significations suivantes :
R représente
a) un atome d'hydrogène, des groupes -F, -Cl, -CF₃, -OCF₃ ou -CN
b) un reste alkyle linéaire ou ramifié (avec ou sans atome de carbone asymétrique) présentant 1 à 20 atomes de carbone, où
b1) un ou plusieurs groupes CH₂ non adjacents et non terminaux peuvent être remplacés par des groupes -O-, -S-, -CO-, -CO-O-, -O-CO-, -O-CO-O- ou -Si(CH₃)₂- et/ou
b2) un ou plusieurs groupes CH₂ peuvent être remplacés par des groupes -CH=CH-, -C≡C-, cyclopropane-1,2-diyle, 1,4-phénylène, 1,4-cyclohexylène ou 1,3-cylopentylène et/ou
b3) un ou plusieurs atomes de H peuvent être remplacés par des groupes F, CN et/ou Cl et/ou
b4) les groupes CH₃ terminaux peuvent être remplacés par un des groupes chiraux (optiquement actifs ou racémiques) : 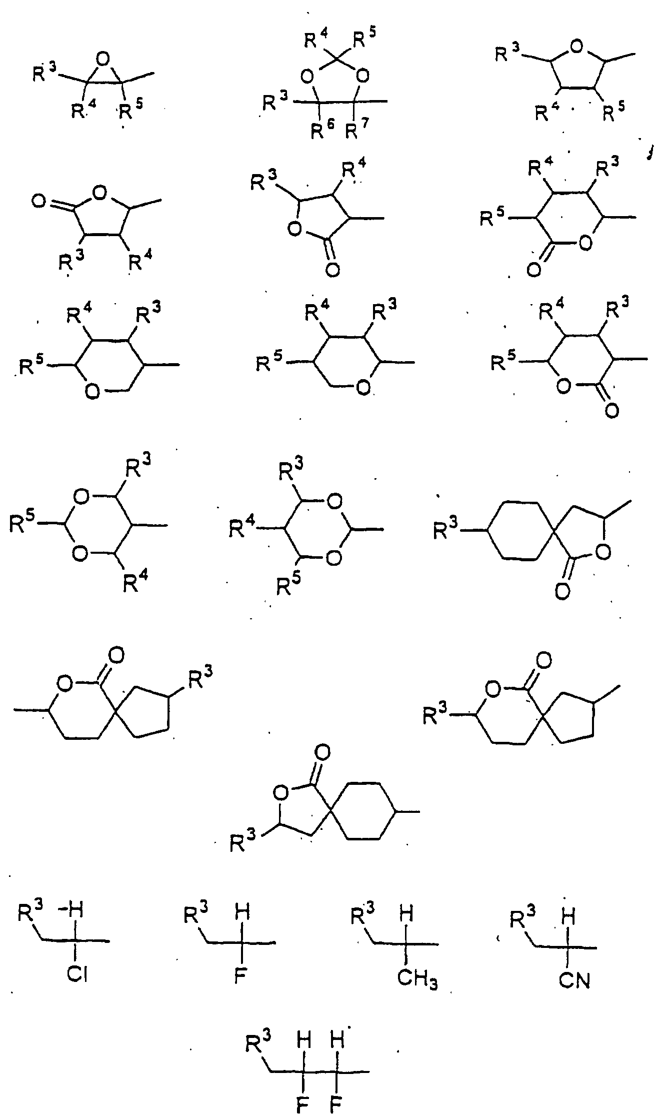
c) B-Rₗ
R³, R⁴, R⁵, R⁶, R⁷ sont identiques ou différents et représentent
a) un atome d'hydrogène
b) un reste alkyle linéaire ou ramifié (avec ou sans atome de carbone asymétrique) présentant 1 à 16 atomes de carbone, où
b1) un ou plusieurs groupes CH₂ non adjacents et non terminaux peuvent être remplacés par des groupes -O- et/ou
b2) un ou plusieurs groupes CH₂ peuvent être remplacés par des groupes -CH=CH-,
c) R⁴ et R⁵ représentent conjointement des groupes -(CH₂)₄- ou -(CH₂)₅- également , lorsqu'ils sont liés à un système oxiranne, dioxolanne, tétrahydrofuranne, tétrahydropyrane, butyrolactone ou valérolactone ;
Rₗ représente
un reste alkyle linéaire ou ramifié, partiellement fluoré ou perfluoré (avec ou sans atome de carbone asymétrique) présentant 1 à 20 atomes de carbone, où
a) un ou plusieurs groupes CH₂ ou CF₂ non adjacents et non terminaux peuvent être remplacés par des groupes -O-, -S-, -CO-, -CO-O-, -O-CO-, -O-CO-O- ou -Si(CH₃)₂- et/ou
b) un ou plusieurs groupes CH₂ ou CF₂ peuvent être remplacés par des groupes -CH=CH-, -C≡C-, cyclopropane-1,2-diyle, 1,4-phénylène, 1,4-cyclohexylène ou 1,3-cylopentylène ;
B représente des groupes
-O-, -S-, - (CH₂)ₙ₋₁-O-, -O-(CH₂)ₙ₋₁, -(CH₂)ₙ₋₁-S-, -S-(CH₂)ₙ₋₁-, -CO-(CH₂)ₙ-, -CO-O-(CH₂)ₙ-, -O-CO-(CH₂)ₙ-, -CO-S-(CH₂)ₙ-, -S-CO-(CH₂)ₙ-, -CS-O-(CH₂)ₙ-, -O-CS-(CH₂)ₙ-, -SO₂-(CH₂)ₙ-, -OSO₂-(CH₂)ₙ-, -CH=CH-, -C≡C-, -(CH₂)ₙ₋₁-, -CH=N-, -N(CₖH₂ₖ₋₁)-, -(CH₂)ₙ-N(CₖH₂ₖ₋₁)-CO-, -(CH₂)ₙ-N(CₖH₂ₖ₋₁)-SO₂-, -O-[(CH₂)ₘ₋₁-O]-(CH₂)ₙ-, -[(CH₂)ₘ₋₁-O]-(CH₂)ₙ- ou une simple liaison ;
m, n sont identiques ou différents, représentent indépendamment l'un de l'autre, un entier de 0 à 15, k est un entier de 0 à 4 et 1 est un entier de 1 à 6 à condition que m+n ≤ 15 ;
M¹, M², M³ sont identiques ou différents et représentent des groupes
-CO-O-, -O-CO-, -CO-S-, -S-CO-, -CS-O-, -O-CS-, -CS-S-, -S-CS-, -CH₂-O-, -O-CH₂-, -CH₂-S-, -S-CH₂-, -CH₂-CH₂-, -CH=CH-, -C≡C-, -CH₂-CH₂-CO-O-, -O-CO-CH₂-CH₂-, -CH=N- ou une simple liaison :
A¹, A², A³, A⁴ sont identiques ou différents et représentent des groupes
1,4-phénylène où un ou plusieurs atomes d'hydrogène peuvent être remplacés par des groupes F, Cl, CH₃, C₂H₅, OCH₃, CF₃, OCF₃ et/ou CN, 1,3-phénylène où un ou deux groupes CH peuvent être remplacés par des atomes de N, pyrazine-2,5-diyle où un ou deux atomes d'hydrogène peuvent être remplacés par des groupes F, Cl et/ou CN, pyridazine-3,6-diyle où un ou deux atomes d'hydrogène peuvent être remplacés par des groupes F, Cl et/ou CN, pyridine-2,5-diyle où un ou plusieurs atomes d'hydrogène peuvent être remplacés par des groupes F, Cl et/ou CN, pyrimidine-2,5-diyle où un ou deux atomes d'hydrogène peuvent être remplacés par des groupes F, Cl et/ou CN, 1,4-cyclohexylène où un ou deux atomes d'hydrogène peuvent être remplacés par des groupes CN et/ou CH₃ et/ou F, (1,3,4)-thiadiazol-2,5-diyle, 1,3-dioxane-2,5-diyle, 1,3-dithian-2,5-diyle, 1,3-thiazol-2,4-diyle où un atome d'hydrogène peut être remplacé par des groupes F, Cl et/ou CN, 1,3-thiazol-2,5-diyle où un atome d'hydrogène peut être remplacé par des groupes F, Cl et/ou CN, thiophène-2,4-diyle où un atome d'hydrogène peut être remplacé par des groupes F, Cl et/ou CN, thiophène-2,5-diyle où un ou deux atomes d'hydrogène pouvant être remplacés par des groupes F, Cl et/ou CN, naphtalène-2,5-diyle, naphtalène-1,4-diyle ou naphtalène-1,5-diyle où pour chacun un ou plusieurs atomes d'hydrogène peuvent être remplacés par des groupes F, Cl et/ou CN et/ou un ou deux groupes CH pouvant être remplacés par des atomes de N, phénanthrène-2,7-diyle ou 9,10-dihydrophénathrène-2,7-diyle où pour chacun un, deux ou plusieurs atomes d'hydrogène peuvent être remplacés par des atomes de F et/ou un ou deux groupes CH peuvent être remplacés par des atomes de N, indane-2,5-diyle, indan-1-one-2,5-diyle, benzothiazol-2,6-diyle, benzothiazol-2,5-diyle, benzoxazol-2,6-diyle, benzoxazol-2,5-diyle, benzofurane-2,5-diyle, benzofurane-2,6-diyle, 2,3-dihydrobenzofurane-2,5-diyle, pipérazine-1,4-diyle, pipérazine-2,5-diyle, 1-alkyl-1-silacyclohexylène-1,4-diyle ou 1,3-dioxaborinane-2,5-diyle ;
a, b, c valent zéro ou un,
à condition que les composés de formule (I) ne puissent contenir pas plus de quatre systèmes cycliques à cinq ou plusieurs chaînons.

2. Afficheur à cristaux liquides à matrice active ferroélectriques selon la revendication 1, **caractérisé en ce que** l'afficheur présente un écart d'électrodes d'au moins 1,5 µm et peut commuter sous une des tensions ≤ 30 V.

3. Afficheur à cristaux liquides à matrice active ferroélectriques selon la revendication 1 ou 2, **caractérisé en ce qu'**on utilise un mélange de cristaux liquides constitué par 3 à 30 constitutants, dont 3 à 25 des constituants sont des composés de formule (I).

4. Utilisation d'un cristal liquide ferroélectrique selon la revendication 1 dans des afficheurs à cristaux liquides à matrice active.
